(19) Europäisches Patentamt / European Patent Office / Office européen des brevets

(11) **EP 3 025 699 A1**

(12) **EUROPÄISCHE PATENTANMELDUNG**

(43) Veröffentlichungstag:
01.06.2016 Patentblatt 2016/22

(51) Int Cl.:
*A61K 8/25* (2006.01)  *C01B 33/02* (2006.01)
*C01B 33/027* (2006.01)  *C01B 33/029* (2006.01)

(21) Anmeldenummer: 14195301.8

(22) Anmeldetag: 28.11.2014

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Evonik Degussa GmbH**
**45128 Essen (DE)**

(72) Erfinder:
• **Lang, Dr. Jürgen Erwin**
**76229 Karlsruhe (DE)**
• **Kröll, Dr. Michael**
**Linsengericht (DE)**
• **Uehlenbruck, Goswin**
**61440 Oberursel (DE)**

(54) **Verwendung von Silicium enthaltenden Partikeln zum Schutz von technischen Materialien vor UV-Strahlung**

(57) Die Erfindung betrifft die Verwendung von Silicium enthaltenden Partikeln zum Schutz von technischen Materialien, wie elektrooptischen Schichten oder elektrooptischen Bauteilen vor elektromagnetischer Strahlung im UV-Bereich und optional im Vis- bis in den IR-Bereich, wobei die Partikel als Primärpartikel mit einer Partikelgröße im Bereich von 1 bis 100 nm vorliegen und optional als Cluster der Primärpartikel vorliegen können. Ein besondere Vorteil der erfindungsgemäßen Verwendung besteht in der Möglichkeit die Absorption der elektromagnetischen Strahlung über die Partikelgröße und die Partikelgrößenverteilung definiert an die interessierende zu absorbierenden Wellenlängenbereich anzupassen. Die Silicium enthaltenden Partikel können als bioverträgliche und biologisch abbaubarer UV-Schutz in technischen Anwendungen und Zusammensetzungen für technische Anwendungen als Formulierungen, wie vorzugsweise in Beschichtungszusammensetzungen, wie Lack verwendet werden.

**Beschreibung**

Die Erfindung betrifft die Verwendung von Silicium enthaltenden Partikeln zum Schutz von technischen Materialien, wie elektrooptischen Schichten oder elektrooptischen Bauteilen vor elektromagnetischer Strahlung im UV-Bereich und optional im Vis- bis in den IR-Bereich, wobei die Partikel als Primärpartikel mit einer Partikelgröße im Bereich von 1 bis 100 nm vorliegen und optional als Cluster der Primärpartikel vorliegen können. Ein besonderer Vorteil der erfindungsgemäßen Verwendung besteht in der Möglichkeit die Absorption der elektromagnetischen Strahlung über die Partikelgröße und die Partikelgrößenverteilung definiert an die interessierende zu absorbierenden Wellenlängenbereich anzupassen. Die Silicium enthaltenden Partikel können als bioverträgliche und biologisch abbaubarer UV-Schutz in technischen Anwendungen und Zusammensetzungen für technische Anwendungen als Formulierungen, wie vorzugsweise in Beschichtungszusammensetzungen, wie Lack verwendet werden.

Silane, insbesondere hochreine Silane, bilden eine wichtige Produktklasse in vielen Anwendungsgebieten, wie Halbleiterindustrie, Bauindustrie, Kosmetikindustrie oder Lichtwellenleiterindustrie.

Anwendungen im Bereich der Elektronik oder Optik bzw. Elektrooptik stellen hohe Anforderungen an die Reinheit dieser Substanzen, insbesondere in Bezug auf deren optische Eigenschaften, wie dem Absorptionsverhalten im Bereich der UV-Strahlung sowie der optischen Dichte (Brechzahl, n) im sichtbaren Spektralbereich.

Als besonders negativ erweisen sich im Bereich der Elektrooptik und Coatings, und hier insbesondere im Bereich des UV Spektralbereichs Kristall-Kontaminationen mit der Eigenschaft, die bspw. im Fall von $TiO_2$ photokatalytisch wirken. Solche photokatalytischen Wirkungen können selbst im unteren %-Bereich äußerst störend in den Coatings-Anwendungen sein und deren Lebensdauer stark einschränken. Ferner sind Materialien, die durch einen hohen Brechungsindex charakterisiert sind kaum verfügbar und teuer.

Es besteht daher eine Bedarf an weiteren UV-Filtern mit sehr guten UV-Schutzeigenschaften und gleichzeitig vorzugsweise einer hohen optischen Dichte bzw. Brechzahl. Es wird angenommen, dass mit solchen UV-Filtern elektrooptische Systeme durch eine Dotierung mit den UV-Filtern in ihrem Eigenschaftsprofil eingestellt werden können.

Es bestand daher die Aufgabe, einen optisch verträglichen Stoff herzustellen, der sich einerseits durch herausragende UV-Schutzeigenschaften auszeichnet und andererseits eine hohe optische Dichte (ausgedrückt in der physikalischen Größe Brechungsindex) besitzt, so dass sich elektrooptische Systeme in einem weiten Bereich einfach durch eine Gehalt oder eine Dotierung mit diesem Stoff in ihrem Eigenschaftsprofil einstellen lassen.

Völlig überraschend und unerwartet hat sich nun gezeigt, dass diese Aufgabe gelöst wird, indem ein Silan-Massenstrom, technisch in einem intensiven Infrarotfeld oder in einer bevorzugten alternative in einer Gasentladung behandelt wird. Der Silan-Massenstrom, der vorzugsweise $SiH_4$ enthält, wird einer Plasmaentladungsanordnung zugeführt und dort in einer beispielhaften Hochspannungs-Pulsentladung zur Reaktion gebracht.

Der neue Ansatz liegt darin, über die spezifische Behandlung des Massestroms die Einstellung der Partikelgrößenverteilung zu ermöglichen. Eine Charakterisierung erfolgt dann über die Partikelgröße, der Clusterparameter der Agglomerate sowie der Partikelgrößenverteilung. Zur Berechnung der Clusterparameter wird bevorzugt die Teilchen-Packungsdichte (Raumerfüllung) herangezogen. Hierzu werden die Primärteilchen in erster Näherung als Kugeln vermessen und in der Elementarzelle ausgezählt. Die dem Fachmann bekannte kubisch raumzentrierte Struktur enthält pro Elementarzelle ($1 + 8·⅛ = 2$) Formeleinheiten, die kubisch flächenzentrierte Zelle enthält ($6·½ + 8·⅛ = 4$) Formeleinheiten und hat damit die größere Packungsdichte. Um die Raumerfüllung zu bestimmen, ist wie folgt vorzugehen (alle Kugeln sind in ihrer Dimension identisch):

Die Kantenlänge der kubischen Zellen beträgt a, das Volumen der Zellen ist jeweils $V=a^3$. Im Falle der raumzentrierten Struktur (CRZ) beträgt der Kugelradius genau ¼ der Raumdiagonalen, im Falle der flächenzentrierten Struktur (CFZ) genau ¼ der Flächendiagonalen. Damit ergeben sich 68 % CRZ bzw. 74 % CFZ als Raumerfüllung. Für den Fall des einfachen kubischen Clusters beträgt die Raumerfüllung weniger als 50 %. Aus diesen Daten lässt sich über die dem Fachmann bekannte Antennenapertur als Herzscher Dipol bspw. aus der lateralen Dimension die Absorption als Funktion der Wellenlänge bestimmen. Für weniger symmetrische Partikelgeometrien ergeben sich dann entsprechende Richtungsabhängigkeiten. Die Partikelgrößenverteilung beeinflusst neben den elektrophysikalischen Eigenschaften unmittelbar die die spektralen Absorptionseigenschaften im UV-Vis-Bereich der Partikelkollektive.

Erfindungsgemäß wurde der Bereich des unerwünschten UV- Spektrums bei gleichzeitig höherer Transparenz im Sichtbaren Bereich bedämpft. Das heißt, dass besonders der Bereich der kurzen Wellenlägen von 400 nm bis 200 nm mit den genannten Clustern/Aggregaten gezielt bedämpft wird. Dabei bewirken die elektrophysikalischen Materialeigenschaften (relative Dielektrizitätskonstante, relative Permeabiltätskonstante) ein dem Hochfrequenztechniker bekannte "Verkürzung der geometrischen Länge der Antenne - was zu einem aktiven Absorptionsverhalten geometrisch kleinerer Primärpartikel und deren Cluster bspw. im UV Zielbereich führt. Der neue Ansatz liegt darin, dass den Massestrom dahingehend zu behandeln, dass er durch eine Partikelgrößenverteilung charakterisiert werden kann, über die die spektralen Absorptionseigenschaften im UV-Bereich und im Vis-Bereich sowie optional im IR-Bereich eingestellt werden können. Hinsichtlich der Wirkung der Gasentladungsbehandlung gehen die Erfinder von einer Begünstigung der Kinetik dahingehend aus, dass plasmachemisch nichtkristallines Silicium, also amorphes Silicium, das selektiv und bevorzugt Siliciumpartikel kleiner 70 nm, bei einer mittleren Partikelgröße von ca. 25 nm gebildet werden, die erfindungsgemäß eine hohe Absorption im UV-A bis UV-B, bevorzugt bis UV-C zeigen (UV-C: 100-280 nm, UV-B: 280 bis 315 nm, UV-A: 315 bis 380 nm). In Abhängigkeit von der mittleren Primärartikelgröße kann die Absorption im UV-C-und UV-B-Bereich gegenüber der Absorption im UV-A-Bereich gesteigert werden. Ferner kann in Abhängigkeit von der mittleren Primärpartikelgröße kann die Absorption im UV-C-, UV-B- und UV-A-Bereich gegenüber der Absorption im Vis-Bereich gesteigert werden.

Der Massestrom ist im erfindungsgemäßen Verfahren einer einfachen Aufarbeitung, wie Filtration, zugänglich, wodurch das Reinprodukt bevorzugt abgezogen wird. Anfallendes Restgas, das neben Monosilan auch $H_2$ enthält, wird einer Separation unterzogen und dabei gewonnenes Monosilan kann zurückgeführt werden. Für die bekannten Prinzipien der Gasentladung und der Plasma-Chemie wird auf die einschlägige Fachliteratur verwiesen, beispielsweise auf A.T. Bell in "Fundamentals of Plasma Chemistry", ed. J.R. Hollahan und A.T. Bell, Wiley, New York (1974).

Die Aufgaben konnten ebenfalls in überraschender Weise gelöst werden, indem Silicium enthaltende Partikel durch Umsetzung von Monosilan oder höheren H-Silanen, wie der Formel MeHn oder $Me_2H_{2n-2}$, wobei Me für Silicium und n für eine ganze Zahl stehen, Halogensilanen, höheren Halogensilanen oder Alkoxysilanen optional mit C oder N oder Ge enthaltenden Spezies in einem thermischen Prozess, und/oder in einem Plasmaprozess umgesetzt werden. Höhere H-Silane oder Chlorsilane werden auch als Polysilane oder Polychlorsilane bezeichnet. Die höheren H-Silane oder höheren Chlorsilane werden vor Ihrer Umsetzung in die Gasphase überführt. Das Halogen kann ausgewählt sein aus Fluor, Chlor, Brom oder Iod, bevorzugt ist Chlor.

Erfindungsgemäß werden die Aufgaben ebenso gelöst durch die erfindungsgemäße Verwendung von Silicium enthaltenden Partikeln, insbesondere von amorphen, insbesondere nicht kristallinen, reinen Silicium enthaltenen Partikeln, indem die Partikel zum Schutz von technischen Materialien vor elektromagnetischer Strahlung im Wellenlängenbereich von 10 bis 2500 nm, insbesondere bis 1500 nm verwendet werden. Bevorzugte technische Materialien umfassen technische Oberflächen oder technischen Bauteilen, besonders bevorzugt elektrooptische Schichten, elektrooptische Bauteile, elektronische Schichten, elektronische Bauteile, technische Gläser, optische Gläser, Klebebänder sowie weitere dem Fachmann geläufige Materialien und Bauteile etc. Erfindungsgemäß kann die Verwendung in der Masse und/oder als Oberflächenbeschichtung erfolgen.

Gegenstand der Erfindung ist somit auch die Verwendung der Silicium enthaltenden Partikel zum Schutz, wie UV-Schutz, von technischen Materialien in der Masse und/oder mittels einer Beschichtung vor elektromagnetischer Strahlung, von technischen Materialien, Oberflächen, Bauteilen, elektrooptische Schichten, elektrooptischer Bauteile, und/oder zur Verwendung als Beschichtung.

Insbesondere werden die Partikel zum Schutz vor einer Zersetzung, Schädigung durch UV-Strahlung oder auch zur Erhöhung der optischen Dichte von Materialien verwendet.

Besonders bevorzugt ist die Verwendung von Silicium enthaltenden Partikeln, die im Wesentlichen amorphe Partikel sind. Als amorph gelten Partikel, die eine Kristallinität von kleiner gleich 2 % aufweisen.

Bevorzugte reine Silicium enthaltende Partikel, insbesondere Primärpartikel, die vorzugsweise in Clustern vorliegen, weisen einen Gehalt von Silicium von größer gleich 90 Gew.-% bis 100 Gew.-% in Bezug auf die Gesamtzusammensetzung an Silicium enthaltenden Partikeln auf, insbesondere beträgt der Gehalt an Silicium größer gleich 55 Gew.-%, bevorzugt beträgt der Siliciumanteil größer 80 Gew.-%, 95 Gew.-% , 95 Gew.-%, 98 Gew.-%, 99 Gew.-%, 99,5 Gew.-%, 99,99 Gew.-%, 99,999 Gew.-% bis 100,0 Gew.-%, und optional weisen die Partikel auf den Siliziumanteil 100 in Gew.-% einen Gehalt an Kohlenstoff und/oder Sauerstoff auf, wobei vorzugsweise die Partikel im Wesentlichen amorph sind.

Der Gehalt an Sauerstoff liegt kleiner 50 Gew.-% bevorzugt kleiner 30 Gew.-% besonders bevorzugt kleiner 10 Gew.-%, abhängig von der Primärpartikelgröße. Der Gehalt richtet sich erfindungsgemäß nach dem Oberflächen zu Volumenverhältnis.

Besonders bevorzugt ist eine Verwendung von Silicium enthaltenden Partikeln, die im Wesentlichen amorph sind und bestehen aus Silicium, Siliciumcarbid, Siliciumnitrid ($Si_3N_4$), SiC-Si, SiGe, SiGe:C oder Gemischen dieser, optional mit einem Gehalt an Sauerstoff, wie Si-O sowie einem möglichen Dotierstoff wie z. B. Selen als Radikalfänger.

Ebenso Gegenstand der Erfindung ist die Verwendung der Silicium enthaltenden Partikel zur Absorption von elektronmagnetischer Strahlung im Wellenlängenbereich von größer gleich 10 nm bis 1100 nm, insbesondere im Wellenlängenbereich von 10 nm bis 450 nm. Bevorzugt werden die Silicium enthaltenden Partikel als UV-Schutz verwendet, vorzugsweise als UV-Schutz im Wellenlängenbereich von 180 bis 400 nm, besonders bevorzugt von 200 bis 380 oder bis 400 nm. Ebenso bevorzugt ist, dass die Silicium enthaltenden Partikel auch vor elektromagnetischer Strahlung im Wellenlängenbereich des extremen UV wie 10 bis 100 nm bzw. bis 120 nm, im fernen UV, von 200 bis 280 nm, im mittleren UV von 280 bis 315 nm, und/oder im nahen UV von 315 bis 380 schützen können sowie je nach Partikelgröße der Primärpartikel von 400 bis 750 nm im Vis-Bereich sowie optional im IR-Bereich oberhalb von 750 nm bis ca. 1500 nm vor elektromagnetischer Strahlung schützen können. Die definierte Absorption in den vorgenannten Bereichen kann spezifisch über den Gehalt der jeweiligen mittleren Primärpartikelgrößen gezielt eingestellt werden.

Ferner können vorzugsweise Silicium enthaltenden Partikel verwendet werden, deren Gehalt größer gleich 40 bis 100 Gew.-% Silicium beträgt und 60 bis 0 Gew.-% eines Gehaltes an Stickstoff, Kohlenstoff und/oder Elementen der CAS-Gruppe IIIA oder VA, bevorzugt Bor, Aluminium, Phosphor, Arsen, Selen und/oder Antimon, und optional Sauerstoff, umfasst, wobei die Primärpartikelgröße von 1 bis 500 nm beträgt, vorzugsweise liegt die Primärpartikelgröße bei 1 bis 80 nm, weiter bevorzugt bei 1 bis 70 nm.

Es hat sich gezeigt, dass sich die spektroskopischen Eigenschaften der amorphen Partikel auch direkt über das Herstellverfahren hinsichtlich der Verteilung der Primärpartikelgrößen und/oder Clusterbildung, sowie optional über einen Zusatz von Kohlenstoff, Stickstoff, Germanium oder weiteren Silanen einstellen lassen.

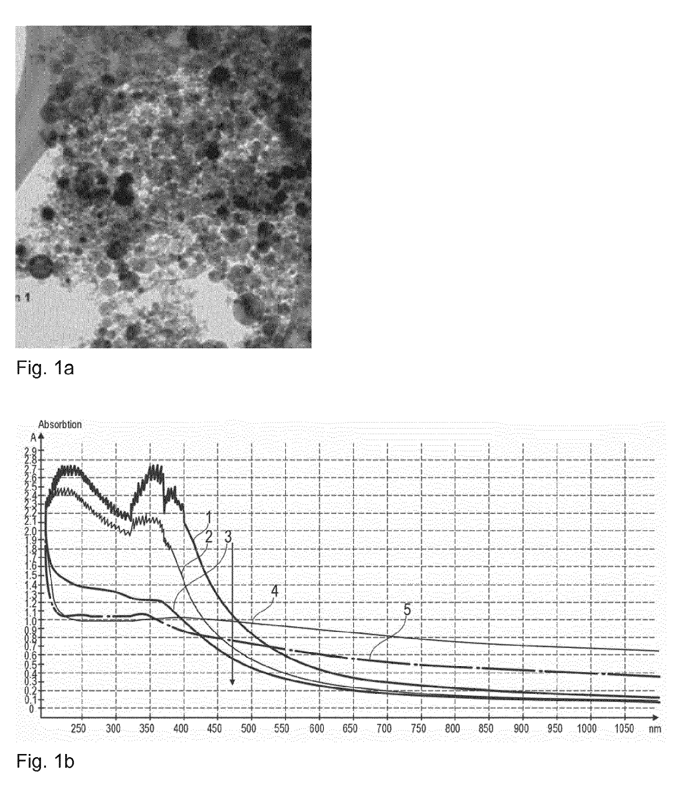

So konnte die Absorption über die Partikelgröße der Silicium enthaltenden Partikel, die Primärpartikel und Cluster der Primärpartikel umfassen, unmittelbar eingestellt werden. Überraschend absorbieren Partikel mit einer Primärpartikelgröße von 5 bis 80 nm und unterschiedlichen mittleren Partikelgrößen von $d_{50}$ um 20, 25, 30 35 und 40 nm im UV- und im Vis-Bereiche deutlich unterschiedlich. Siliciumpartikel mit einer Partikelgröße der Primärpartikel von im Mittel 25 bis 30 nm, insbesondere mit $d_{90}$ 10 bis 50 nm, absorbieren deutlich stärker im UV-Bereich als im Vis-Bereich, während Partikel mit einer Primärpartikelgröße von im Mittel von 35 bis 40 nm, insbesondere mit $d_{90}$ 10 bis 70 nm, vorzugsweise 10 bis 50 nm, im UV/Vis-Bereich vergleichbar stark absorbieren (Figur 1b).

Gegenstand der Erfindung ist auch die Verwendung von Silicium enthaltenden Partikel mit einer Partikelgröße ausgewählt aus: a) mittlere Primärpartikelgröße von im Mittel $d_{50}$ = 1 nm bis 200 nm aufweisen, insbesondere weisen sie im Mittel Primärpartikel von 5 bis 100 nm auf, und/oder b) mittlere Primärpartikelgröße von im Mittel $d_{50}$ = 1 bis 10 nm, und/oder c) mittlere Primärpartikelgröße von im Mittel $d_{50}$ = 16 bis 40 nm, sowie Gemischen umfassend a, b und/oder c. Die optischen Eigenschaften der Gemische können spezifisch über den Gehalt der jeweiligen mittleren Primärpartikelgröße eingestellt werden.

Vorzugsweise weisen die amorphen Silicium enthaltenden Partikel einen Primärpartikelgröße von im Mittel gemäß Beispiel-TEM P6: 20 nm, 25 m, 30 nm oder 40 nm, jeweils unabhängig mit einer geringen Streuung von +/- 10 nm, insbesondere +/- 5 nm für $d_{90}$ auf.

Die Cluster der Primärpartikel können 30 bis 400 nm groß sein, wobei die Cluster übliche Größen etwa von 150 nm mit plus/minus 50 nm oder alternativ von 50 bis 100 nm aufweisen.

Erfindungsgemäße Silicium enthaltende Partikel mit Primärpartikeln mit einer mittleren Partikelgröße der Primärpartikel von 10 bis 30 nm, wie vorzugsweise um $d_{50}$ = 13,2 nm und vorzugszugsweise $d_{20}$ = 7,2 nm und $d_{90}$ = 23,5 nm, weisen im Wellenlängenbereich von 180 bis 1000 nm eine Transparenz von kleiner 40 % (Absorption größer gleich 0,6), insbesondere bei 180 bis 700 nm eine Transparenz von kleiner gleich 20 % (Absorption größer gleich 0,8), vorzugsweise zudem von 180 bis 475 nm eine Transparenz kleiner gleich 5 % (Absorption größer 0,95) auf, wobei vorzugsweise die Primärpartikel Cluster einer mittleren Clustergröße von größer gleich 50 nm bilden und amorph sind.

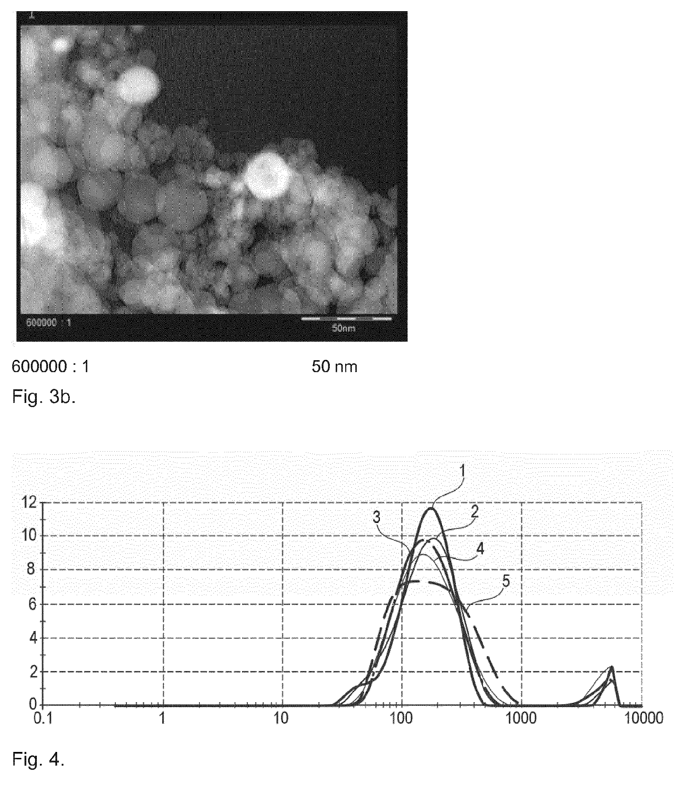

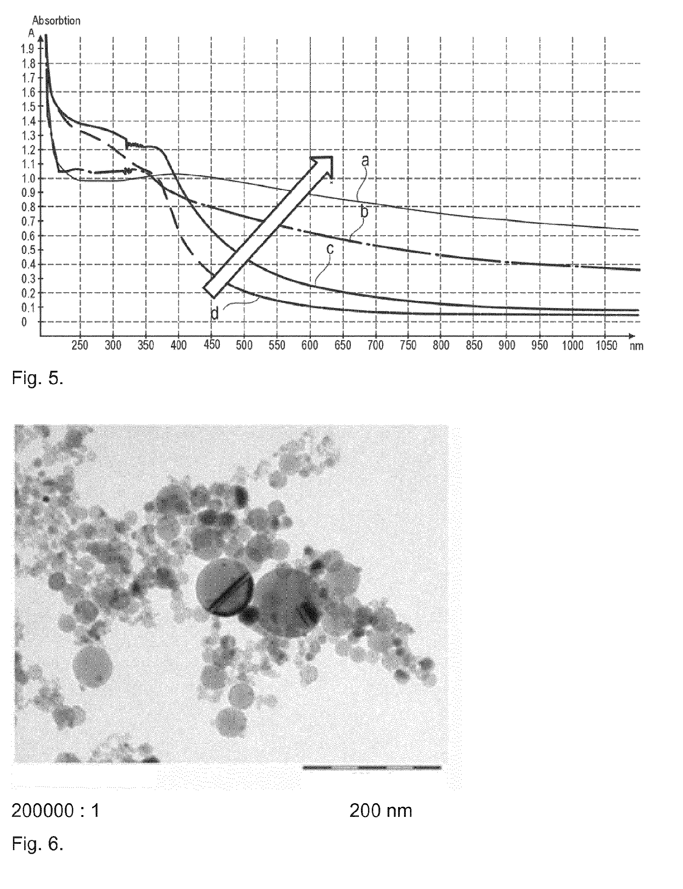

Alternative erfindungsgemäße Silicium enthaltende Partikel mit Primärpartikeln mit einer mittleren Partikelgröße der Primärpartikel von 35 bis 40 nm, weisen im Wellenlängenbereich von 180 bis 400 nm eine Transparenz von kleiner 40 % (Absorption größer gleich 0,6), insbesondere bei 180 bis 350 nm eine Transparenz von kleiner gleich 0 % (Absorption größer gleich 1,0), vorzugsweise zudem von 180 bis 300 nm eine Transparenz kleiner gleich 0 % (Absorption größer 1,2) auf, wobei vorzugsweise die Primärpartikel Cluster mit einer mittleren Clustergröße von 30 bis 400 nm, insbesondere um 150 nm mit plus/minus 50 nm bilden und amorph sind. So zeigt Figur 4 die Größenverteilung der Cluster und Figur 5 das UV-Vis-Absorptionsverhalten einer Referenzprobe sowie von Si unterschiedlicher Primärpartikel- und unterschiedlicher Clustergrößen.

Ebenfalls Gegenstand der Erfindung ist die Verwendung von Silicium enthaltenden Partikeln mit 40 bis 100 Gew.-% Silicium und auf 100 Gew.-%, d. h. 60 bis 0 Gew.-%, eines Gehaltes an Stickstoff, Kohlenstoff und optional Sauerstoff. Die SiC enthaltenden Partikel sind vorzugweise transparent. Gemäße einer Alternative weisen die Partikel zu 60 bis 100 Gew.-% Silicium auf und auf 100 Gew.-% weisen sie einen Gehalt an Kohlenstoff und optional Sauerstoff auf. Diese Partikel sind vorzugsweise transparent. Insbesondere umfassen die Siliciumpartikel bis zu 40 bis 50 Mol-% Silicium und 40 bis zu 50 Mol.-% einen Gehalt an Kohlenstoff und optional zusätzlich Sauerstoff. Besonders bevorzugt sind SiC-Partikel mit optional einem Gehalt von weniger als 10 Mol-% an Si-O. Gemäß einer weiteren Alternative weisen die Partikel zu 35 bis 50 Gew.-% Silicium auf und auf 100 Gew.-%, d. h. zu 50 bis 65 Gew.-% weisen sie einen Gehalt an Stickstoff und optional Sauerstoff auf. Bevorzugt sind Siliciumnitrid-Partikel, die vorzugsweise in einem Coating im Vis-Bereich transparent sind. Insbesondere umfassen die Siliciumpartikel zu 40 Gew.-% Silicium und auf 100 Gew.-% einen Gehalt an Stickstoff und optional Sauerstoff, besonders bevorzugt sind $Si_3N_4$-Partikel optional mit einem Gehalt an Si-O.

Vorzugsweise beträgt der Gehalt an Silicium in der Gesamtzusammensetzung der Silicium enthaltenden Partikeln oder in den jeweiligen einzelnen Partikeln größer gleich 70 bis 99,9999 Gew.-%, insbesondere 80 bis 99,9999 Gew.-%, vorzugsweise 90 bis nahezu 100 Gew.-%, besonders bevorzugt 95 bis nahezu 100 Gew.-

%, besonders bevorzugt größer gleich 98,5 Gew.-% an Silicium sowie optional zusätzlich mindestens Kohlenstoff und/oder Sauerstoff.

Gleichfalls können Silicium enthaltende Partikel verwendet werden, die im Wesentlichen bestehen aus amorphen, reinen Silicium Partikel oder auch amorphen, reinen Siliciumcarbid, Siliciumnitrid und/oder Silicium-Germanium Partikel vorgenannter Primärpartikelgröße oder Gemischen dieser.

Erfindungsgemäß wird ein großtechnisches, industrielles, vorzugsweise kontinuierliches Verfahren zur Herstellung von Silicium enthaltenden Partikeln, umfassend Primärpartikel und optional Cluster der Primärpartikel bereitgestellt.

Die Umsetzung, insbesondere umfassend die Zersetzung und Bildung der Partikel und der Cluster, kann bei Temperaturen ab 150 °C, bevorzugt ab 400 bis 1500 °C zur Herstellung amorpher Pulver erfolgen. Zur Herstellung amorpher Partikel werden kurze Kontaktzeiten, vorzugsweise bei Temperaturen unter 1300 °C, gewählt. Alternativ kann die Bildung amorpher Primärpartikel bei Temperaturen um 1300 °C bevorzugt kleiner gleich 1100 °C erfolgen. Die Abscheidung der Partikel erfolgt in einer kühleren Zone des Reaktors. Bevorzugte Kontaktzeiten liegen von 10 bis 600 Millisekunden.

Im Rahmen der Erfindung werden die Begriffe *"Kontaktzeit"* und *"Verweilzeit"* gleichbedeutend verstanden.

So benötigen übliche konventionelle Verfahren, die auf einer Umsetzung von: $SiCl_4$ basieren 30 kW/kg und mehr. Die erfindungsgemäßen Verfahren beruhen vorzugsweise auf einer Umsetzung von Monosilan mit einem Energiebedarf von weniger als 10 kW/kg besonders bevorzugt um 5 kWh/kg. Des Weiteren wird für die Umsetzung im (kalten) Plasma der Energiebedarf weiter reduziert auf kleiner 4 kW/kg.

Als besonders bevorzugt gelten Silicium enthaltenden Partikel in denen weniger als 40 % der Partikel eine Abweichung von der mittleren Korngröße $d_{50}$ und weniger als 25 % eine Abweichung von größer gleich 50 % von der mittleren Korngröße $d_{50}$ aufweisen.

Bevorzugt sind weiterhin Silicium enthaltenden Partikel deren Primärpartikel einen mittleren Durchmesser $d_{50}$ (gemessen durch TEM-Auswertung; TEM = Transmissions-Elektronenmikroskop) im Bereich von 5 bis 80 nm, vorzugsweise von 20 bis 50 nm, aufweisen und, die vorzugsweise als aneinandergewachsene Cluster vorliegen. Die Cluster könne auch als Agglomerate bezeichnet werden, wobei vorliegend unter einem Cluster aneinandergewachsene bzw. aneinander geschmolzene Primärpartikel verstanden werden. So können die Primärpartikel Cluster ausbilden, in denen mindestens zwei Primärpartikel an ihren Oberflächen aneinander geschmolzen sind. Diese Cluster können als lineare Ketten, in Form von Drähten oder auch verzweigt im 3-Dimensionalen Raum vorliegen.

Ob die Teilchen sich sphärisch oder in der Form von Whiskern ausbilden hängt unter anderem von der $H_2$-Konzentration bei der Darstellung ab. Je nach Temperaturprofil, Reinheit (Anwesenheit von metallischen Elementen bspw. Selen (Se) im Gasstrom), Verdünnungsgas (Konzentration, Strömungsgeschwindigkeit) Herstellbedingungen können Primärpartikel isoliert werden oder überwiegend Primärpartikel, die zu Clustern agglomeriert sind, erhalten werden. So können bei einer verdünnten Prozessführung überwiegend Primärpartikel isoliert werden und bei hoher Prozessgaskonzentration und/oder hoher Temperatur, bspw. um 1500 °C in einer bevorzugten Variante verwachsene Cluster isoliert werden.

Gegenstand der Erfindung ist auch die Verwendung von Clustern der Primärpartikel als Schutz vor elektromagnetischer Strahlung. Dies schließt neben dem Gebiet des UV-VIS-IR das daran anschließende Terraherz, sowie das Hoch und Höchstfrequenzgebiet, und den Bereich der Radiowellen bis hin zu Langwellen (>100 Herz) mit ein.

Entsprechend einer weiteren Alternative umfassen die Silicium enthaltenden Partikel vorzugsweise Primärpartikel, die im Wesentlichen sphärisch sind. Die mittlere Sphärizität, definiert als Aspektverhältnis der Durchmesser der im 90° Winkel aufeinander stehenden Durchmesser ist vorzugsweise kleiner gleich 1,6, vorzugsweise kleiner gleich 1,4 bis größer gleich 0,9, vorzugsweise von 0,95 bis 1,2. Das Aspektverhältnis nahe der Kugelform erlaubt eine ideale Korrelation der UV Absorption an die Primärpartikelgröße sowie eine gute Homogenisierbarkeit. Ferner können die Silicium enthaltenden Partikel Primärpartikel und Cluster der Primärpartikel umfassen, insbesondere weisen die Cluster eine Größe von 10 nm bis 3 μm auf, vorzugsweise von 100 nm bis 3 μm, weiter bevorzugt von 1 μm bis zu 6 μm. Bevorzugte Silicium enthaltenden Partikel umfassen Silicium (Si) oder ggf. SiC, SiGe, SiN-Verbindungen, von größer gleich 99,9999 Gew.-% an Silicium. Optional weisen die SiC, SiGe, SiN-Verbindung eine entsprechenden Gehalt an diesen Verbindungselementen auf. Bevorzugt liegt der Silizium- Gehalt bei größer gleich 94,99 Gew.-%, besonders bevorzugt bei größer gleich 97,999 Gew.-%. Ebenso bevorzugt sind Partikel umfassend Silicium-Stickstoff-, Silicium-Kohlenstoff- und/oder Silicium-Germanium-Verbindungen, wie Siliciumnitrid, Siliciumcarbid, Siliciumcarbid in einer Siliciummatrix. Die Silicium-Stickstoff-, Silicium-Germanium-, Silicium-Kohlenstoff-Verbindungen können auch partikulär in einer Matrix von im Wesentlichen reinem Silicium vorliegen. Vorzugsweise liegen die Silicium enthaltenden Partikel im Wesentlichen ohne eine äußere Matrix oder Beschichtung vor, die auch eine passivierende Oxidschicht umfassen kann. Nach einer Alternative liegen die Silicium enthaltenden Partikel mit einer Siliciumdioxid-Zone (Core-shell) von typisch 1 nm vor. Bevorzugt bzw. im Idealfall umfaßt die Zone eine Monolage auf der Oberfläche.

Dabei ist es erfindungsgemäß besonders bevorzugt, wenn die Silicium enthaltenden Partikel hochrein sind, insbesondere höchstrein. Als hochrein gelten die Partikel, wenn Silicium mit einem Gehalt größer gleich 99,99 Gew.-% in der Gesamtzusammen-setzung vorliegt, bevorzugt mit einem Gehalt von größer gleich 99,999 Gew.-%. Als höchstrein gelten die Silicium enthaltenden Partikel mit einem Gehalt von größer gleich 99,9999 Gew.-% Silicium in der Gesamtzusammensetzung. Wobei eine Siliciumdioxid Core-Shell außer Betracht bleibt.

Die Verunreinigungen in den jeweiligen Edukten und Verfahrensprodukten werden mittels dem Fachmann bekannter Probenaufschlussverfahren bspw. durch Nachweis im ICP-MS (Analytik für die Bestimmung der Spurenverunreinigung) bestimmt.

In den erfindungsgemäßen Partikeln weisen die Primärpartikel und optional die Cluster abhängig von ihrer Größe in der Gesamtzusammensetzung einen Gehalt von kleiner gleich 2 Atom-%/cm3, vorzugsweisen kleiner gleich 2000 ppm Sauerstoff auf, vorzugsweise kleiner gleich 1000 ppm, insbesondere kleiner 10 ppm. Die Analyse erfolgt neben ICP-MS und HP-GD-MS (High Purity Glow Discharge Mass Spectroskopy) oder bevorzugt mittels Neutronenaktivierungsanalyse (NAA). Die NAA ist eine hochempfindliche physikalische Technik der analytischen Chemie zur qualitativen und quantitativen Spurenanalyse, bei der die zu untersuchende Probe mit Neutronen (oder anderen Teilchen) beschossen wird.

Bei der NAA wird eine Probe von nur wenigen Milligramm (oder fallweise wenige μg) dem Neutronenstrom (eines Kernreaktors) ausgesetzt. Die Neutronen reagieren mit den Kernen der Probe und machen aus den stabilen Isotopen radioaktive Isotope, deren Massenzahl eins höher ist als die Massenzahl des stabilen Isotops. Bei diesem ersten Kernprozess wird ein promptes γ-Quant ausgesandt, dessen Energie man messen kann und das Auskunft über den Ausgangskern gibt. In den meisten Untersuchungen wird aber erst der Zerfall des entstandenen radioaktiven Kerns zur Analyse verwendet. Es zerfällt anschließend mit der für ihn typischen Halbwertzeit unter Aussendung eines Betateilchens und charakteristischer Gammastrahlung, die in einem Gammaspektrometer untersucht wird. Auf diese Weise sind praktisch alle vorkommenden Elemente in einer Probe quantitativ und qualitativ nachweisbar. Aus den bekannte Eigenschaften der Atomkerne lassen sich neben den Gehalt der Elemente, sogar ihrer Isotope bestimmen. Die Messungen können bspw. am Berlin Neutron Scattering Center durchgeführt werden.

Der Gehalt an Verdünnungsgasen, wie Xenon, Argon, Krypton, oder auch Stickstoff beträgt in der Gesamtzusammensetzung der Partikel weniger 1% Atome/cm3, insbesondere kleiner gleich 1000 ppm-Gew., 10 ppm-Gew., 1 ppb-Gew bis zur Nachweisgrenze, bspw. bis 1 ppt-Gew.

Die Nachweisgrenzen für die Bestimmung von Xenon (Xe), Argon (Ar), Krypton (Kr), oder auch Stickstoff durch Neutronenaktivierung bei einer Bestrahlzeit von 1 Stunde bei einer sogenannten Flussdichte an thermischen Neutronen von 10Exp 14 cm$^{-2}$ s-1 liegen bei ca.

10$^{-8}$ g (Ne, Xe) ca. 10$^{-9}$ g (Kr) ca. 10$^{-10}$ g (Ar) und ca. 10$^{-5}$ g für Sauerstoff (O$_2$) Die Nachweisgrenze kann damit insbesondere für kleine Probenmengen kleiner gleich 1000 ppm-Gew., 10 ppm-Gew., 1 ppb-Gew bzw. bis zur Nachweisgrenze, bspw. bis 1 ppt-Gew. betragen.

Zur Herstellung der hoch- oder höchstreinen Silicium enthaltenden Partikeln wird ein hoch- bis höchstreines Silan, wobei die Definition von rein bis höchstreines Silane mit Halbleiterqualität verwendet werden, wie monomeres und/oder polymeres Monosilan, H-Silan und/oder Chlorsilan eingesetzt, insbesondere der allgemeinen Formel I, II und/oder III oder eine Mischung der enthaltend, wie höchstreines Tetrachlorsilan, höchstreines Trichlorsilan und/oder höchstreines Dichlorsilan, vorzugsweise mit einem Gehalt an einem Silan von 80 bis 99,9999999 Gew.-%, ad 100 Gew.-% ggf. Polysilane, und mit einer Gesamtverunreinigung von kleiner gleich 100 Gew.-ppm bis 0,001 Gew.-ppt als hochreines Silan, vorzugsweise kleiner 50 Gew.-ppm bis 0,001 Gew.-ppt als höchstreines Silan, bevorzugt kleiner gleich 40 Gew.-ppm bis 0,001 Gew.-ppt an Gesamtverunreinigung mit den nachstehend genannten Elementen. Alternativ kann statt einem einzelnen Silan auch eine Mischung von Silanen eingesetzt werden, sofern sie dem vorgenannten Anforderungsprofil an den Gehalt des Silans entspricht.

Gegenstand der Erfindung ist auch die Verwendung von Silicium enthaltenden Partikeln umfassend a) Primärpartikel einer Primärpartikelgröße von 1 nm bis 500 nm, insbesondere weisen sie Primärpartikel von 3 bis 100 nm, vorzugsweise von 5 bis 80 nm auf. Erhalten sind diese Primärpartikelgrößen vorzugsweise über ein Plasma-Verfahren. Des Weiteren ist es bevorzugt, wenn die Partikel zugleich b) eine mittlere Primärpartikelgröße von im Mittel $d_{50}$ = 5 nm, 10 nm, 16 nm, 20 nm, 25 m, 30 nm oder 40 nm aufweisen, insbesondere jeweils unabhängig mit einer geringen Streuung bevorzugt kleiner +/- 25 nm für $d_{90}$, im Fall der 40 nm Partikel, wobei der Anteil der Primärpartikel die vorzugsweise als Cluster vorliegt dabei nicht berücksichtigt sind.

Alternativ weisen Primärpartikel eine Partikelgröße von 10 bis 50 nm auf, mit +/- 35 nm bei 50 nm, insbesondere +/- 25 nm. Primärpartikel, die über ein auf Infrarotstrahlung basierenden bspw. Free-Space-Reaktor- Verfahren zugänglich sind weisen in der Regel Primärpartikel vorzugsweise Cluster der Primärpartikel mit Partikelgrößen von 100 bis 350 nm auf und. Vorzugsweise weisen die erfindungsgemäßen Cluster eine Größe von 10 nm bis 3 μm auf, insbesondere 100 nm bis 3 μm, 150 nm bis 400 nm.

Dabei ist es bevorzugt wenn größer gleich 70 Gew.-% der Primärpartikel als Cluster vorliegen, vorzugsweise größer 80 Gew.-%, bevorzugt größer 85 Gew.-% bis 100 Gew.-%, sowie größer 90, 95, 98, 99,5 Gew.-%.

Je nach Primärpartikelgröße weisen die Silicium enthaltenden Partikel eine hellgelbe, orange oder hellbraune Farbe auf und könne bei daher bei Bedarf auch als Pigmente in Lack-Produkten eingesetzt werden. Hier zeigt sich die besonders überraschende Eigenschaft, nämlich der des gleichzeitigen UV-Schutzes und der holzähnlichen Farbe. Durch die angenehme holzähnliche warme Eigenfarbe werden die Silicium enthaltenden Partikel aufgetragen auf das Holz nicht als störend und unangenehm empfunden, wie es bei bekannten weißen UV-Filtern der Fall ist. Die erfindungs-gemäßen Partikel können ferner eine BET-Oberfläche von größer gleich 40 m$^2$/g aufweisen.

Gleichfalls Gegenstand der Erfindung ist die Verwendung von Partikeln mit einer mittleren Primärartikelgröße $d_{50}$ von 1 bis 5 nm, und insbesondere einer mittleren Clustergröße von 50 bis 100 nm, die wie das Beispiel P4 zeigt im Mittel eine doppelt so große Absorption im Wellenlängenreich von 200 bis 400 nm aufweisen im Vergleich zur Absorption im Wellenlängenbereich von 500 bis 750 nm. Die Fig. 6 zeigt eine TEM Aufnahme der Partikel aus dem Beispiel P4.

Entsprechend einer besonders bevorzugten Ausführungsform werden Silicium enthaltende Partikel verwendet, die insbesondere amorph sind, wobei die Partikel die nachfolgende Absorptionscharakteristika elektronmagnetischer Strahlung aufweisen.

a) Mit einer Primärpartikelgröße von 10 bis 50 nm, insbesondere mit einer mittleren Partikelgröße von $d_{50}$ um 35 nm, ist die Absorption in Wellenlängenbereich von 250 bis 400 im Vergleich zur Absorption im Wellenlängenbereich von 400 bis 750 nm mit einer Abweichung von +/- 40 %, insbesondere +/- 30 %, vorzugsweise +/- 20%, bevorzugt +/-10%, und/oder

b) mit einer Primärpartikelgröße von 10 bis 50 nm, insbesondere mit einer mittleren Partikelgröße von $d_{50}$ um 25 nm, ist das Verhältnis der Absorption in Wellenlängenbereich von (i) 250 bis 400 im Vergleich zur Absorption bei einer Wellenlänge von (ii) 550 nm etwa (i) zu (ii) von 2 : 1 bis 8 : 1, insbesondere 2 : 1 bis 6 : 1, vorzugsweise 3 : 1 bis 6 : 1, mit einer Abweichung von +/- 40 %, vorzugsweise +/- 30 %, bevorzugt +/- 20 %, besonders bevorzugt von +/- 10%, und/oder

c) mit einer Primärpartikelgröße von 10 bis 50 nm, insbesondere mit einer mittleren Partikelgröße von $d_{50}$ um 25 nm, ist das Verhältnis der Absorption in Wellenlängenbereich von (i) 250 bis 400 im Vergleich zur Absorption bei einer Wellenlänge von (ii) 500 nm bei etwa (i) zu (ii) von 1,5 : 1 bis 8 : 1, insbesondere 1,8 : 1 bis 5 : 1, bevorzugt von 2 : 1 bis 4 : 1, mit einer Abweichung von +/- 30 %, insbesondere bevorzugt +/- 20%, besonders bevorzugt +/- 10%, und/oder

d) mit einer Primärpartikelgröße von 10 bis 50 nm, insbesondere mit einer mittleren Partikelgröße von $d_{50}$ um 25 nm, ist das Verhältnis der Absorption in Wellenlängenbereich von (i) 250 bis 350 nm im Vergleich zur Absorption bei einer Wellenlänge von (ii) 450 nm etwa (i) zu (ii) von 2 : 1 bis 4 : 1 mit einer Abweichung von insbesondere +/- 30 %, bevorzugt +/- 20%, besonders bevorzugt +/-10%, und/oder

e) mit einer Primärpartikelgröße von 10 bis 50 nm, insbesondere mit einer mittleren Primärpartikelgröße von $d_{50}$ um 25 nm, ist das Verhältnis der Absorption bei einer Wellenlänge von (i) 250 nm im Vergleich zur Absorption bei einer Wellenlänge von (ii) 450 nm bei etwa (i) zu (ii) von 2 : 1 bis 4 : 1, mit einer Abweichung von insbesondere +/- 30 %, bevorzugt +/- 20 %, besonders bevorzugt +/-10 %, und/oder

f) mit einer Primärpartikelgröße von 10 bis 50 nm, insbesondere mit einer mittleren Partikelgröße von $d_{50}$ um 25 nm, ist das Verhältnis der Absorption bei einer Wellenlänge von (i) 350 nm im Vergleich zur Absorption bei einer Wellenlänge von (ii) 450 nm bei etwa (i) zu (ii) von 2 : 1 bis 3 : 1, mit einer Abweichung von insbesondere +/- 30 %,bevorzugt +/-20 %, besonders bevorzugt +/-10 %).

g) wobei die vorgenannten Werte insbesondere für reine, hochreine bis höchstreine und im wesentlichen amorphe Silicium enthaltende Partikel mit einem Siliciumgehalt von größer gleich 98 Gew.-% und optional einem Gehalt an Kohlenstoff und/oder Sauerstoff erzielt werden, bevorzugt ist ein Gehalt an Silicium von größer gleich 99,5 Gew.-% und optional zusätzlich Sauerstoff ad 100 Gew.-% in der Gesamtzusammensetzung. Die optischen Eigenschaften können gezielt über die Gehalte der Fraktionen der jeweiligen mittleren Primärpartikelgrößen eingestellt werden.

Gegenstand der Erfindung ist gleichfalls die Verwendung von Silicium enthaltenden Partikeln, insbesondere amorphen und im Wesentlichen Silicium enthaltenden Partikeln einer Primärpartikelgröße von 1 bis 100 nm, zur Erhöhung der Brechzahl eines Materials, wie eines elektrooptischen Materials, einer elektrooptischen Schicht.

Ebenfalls Gegenstand der Erfindung ist die Verwendung von Silicium enthaltenden Partikel ausgewählt aus Partikeln mit einem Gehalt an reinem Silicium und SiC mit Silicium 70 bis 90 Gew.-% und 10 bis 30 Gew.-% Kohlenstoff sowie reinem SiC zur Erhöhung der Brechzahl einer Formulierung oder eines Materials.

Gegenstand der Erfindung ist auch die Verwendung von Silicium enthaltenden Partikel ausgewählt aus SiC-Partikeln und Partikeln mit einem Gehalt an Silicium und Kohlenstoff mit Silicium 70 bis 90 Gew.-% und 10 bis 30 Gew.-% Kohlenstoff, wobei die Partikel eine Brechzahl (n): i) von größer gleich 2,5 bis kleiner gleich 1000 nm, ii) von größer gleich 2,75 bis kleiner gleich 500 nm, und/oder iii) von größer gleich 3,0 bis kleiner gleich 230 nm, und/oder iv) von größer gleich 3,5 bis kleiner gleich 50 nm aufweisen, insbesondere für im Wesentlichen reine, amorphe Siliciumcarbid Partikel mit einer Primärpartikelgröße von im Mittel 1 bis 40 nm.

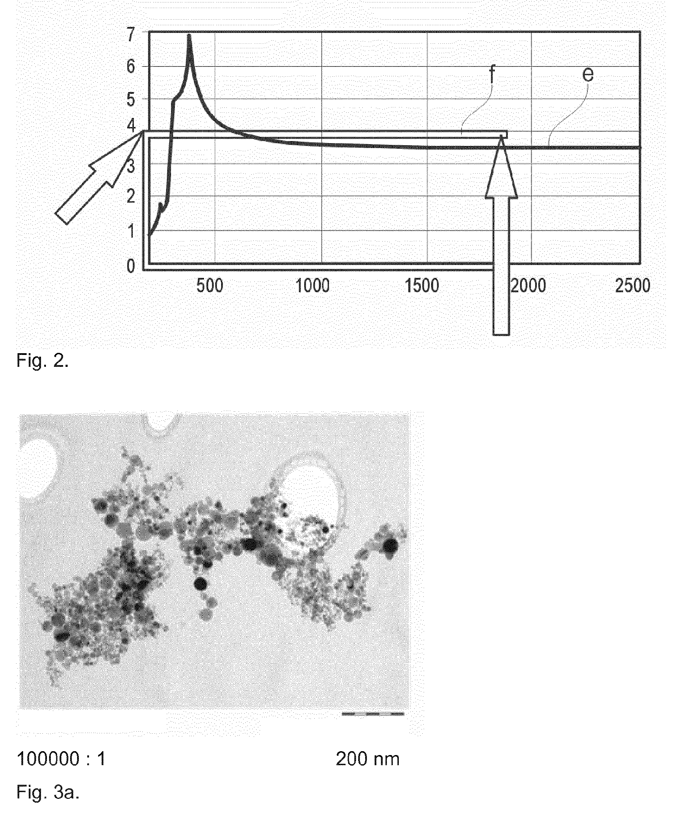

Dabei können Silicium enthaltende Partikel, insbesondere ausgewählt aus Partikeln mit einem Gehalt an Silicium größer 90 bis 100 Gew.-%, vorzugsweise größer gleich 91 bis 100 Gew.-%, vorzugsweises größer gleich 95, 98, 99,5, 99,99 bis 100 Gew.-%, eine Brechzahl (n) von größer gleich 3 bei im Wellenlängenbereich von 500 bis 2500 nm und/oder von größer gleich 4,0 bei einer Wellenlänge von 200 bis 500 nm aufweisen, insbesondere bei etwa 280 bis 400 nm. Bevorzugt weisen die Partikel, insbesondere reine und amorphe Silicium Partikeleine Brechzahl (n) von größer gleich 5 bis 7 im Wellenlängenbereich von etwa 280 bi 400 nm auf, besonders bevorzugt von 6 bis 7. Vorzugsweise weisen im Wesentlichen reine, amorphe Silicium Partikel mit einer Primärpartikelgröße von im Mittel 20 bis 40 nm diese Brechzahlen auf, vorzugsweise von 250 bis 400 nm. Die Brechzahlen erlauben somit auch eine Qualitätskontrolle der Reinheit der hergestellten Silicium enthaltenden Partikel. Figur 2 stellt die Brechzahl von Silicium in Abhängigkeit von der Wellenläng ([nm]) dar.

Gegenstand der Erfindung ist auch die Verwendung von Silicium enthaltenden Partikeln umfassend Primärpartikel von 1 bis 100 nm und optional Cluster dieser Primärpartikel, mit einem Gehalt an Silicium von größer gleich 90 Gew.-%, insbesondere größer gleich 95 Gew.-% bis 100 Gew.-% als biologisch verträglicher UV-Schutz und/oder als biologisch abbaubarer UV-Schutz. Ein weiterer Vorteil der erfindungsgemäßen Partikel ist ihre Verwendbarkeit als anorganischer UV-Schutz, Brechzahl erhöhendes Material und optional Kratzschutz vermittelndes Material.

Weiter ist es bevorzugt, wenn die Partikel eine Core-Shell mit einem Gehalt an Sauerstoff aufweisen, wobei die Partikel in diesem Fall vorzugsweise einen Gehalt an Si-O und/oder Si-OH oder anderweitig mit reaktiven Gruppen funktionalisierbare Verbindungen aufweisen. Partikel mit einer Core-Shell, die auch definierte im Verfahren hergestellt werden kann sind einer Silanisierung und damit einer weiteren Modifizierung und Anbindung oder Einbindung in anderen Materialien zugänglich.

Des Weiteren können nach einer Alternative die Silicium enthaltenden Partikel, die als Primärpartikel und optional Cluster der Primärpartikel vorliegen, und ggf. Siliciumcarbid, Siliciumnitrid, Silicium-Germanium enthalten zusätzlich mit einem Elektronenakzeptor oder Elektronendonator dotiert werden. Zur Dotierung wird während der Herstellung mindestens ein unter den Reaktionsbedingungen reaktives Gases, wie einem Legierungsgas, zugegeben, wie Diboran. Dabei ist es weiterbevorzugt, wenn die Partikel von Zellen, wie den Hautzellen oder Körperzellen abgebaut werden können. So kann der Körper vorzugsweise die amorphen Siliciumpartikel sukzessive zu $SiO_2$ in der wässrigen Körperflüssigkeit umwandeln, resorbieren und abbauen. Wobei die Partikel als im wesentlichen sphärische Partikel oder auch als plättchenförmige Partikel mit einer Schichtdicke von 1 bis 80 nm, insbesondere 20 bis 45 nm, vorliegen können. Als plättchenförmige Partikel können die Partikel vorliegen, wenn die Abscheidung der Partikel auf einer kalten Fläche erfolgt oder die Partikel in eine Folie eingebracht bspw. eingewalzt werden.

Gegenstand der Erfindung ist auch ein Verfahren zur Herstellung von Silicium enthaltenden Partikeln, sowie Silicium enthaltende Partikel erhältlich nach diesem Verfahren, indem mindestens eine gasförmige oder bei erhöhter Temperatur gasförmige Siliciumverbindung, b) optional in Gegenwart mindestens eines unter den Reaktionsbedingungen reaktiven Gases oder einer Mischung von reaktiven Gasen, c) in Gegenwart eines Verdünnungsgases in einer im Wesentlichen sauerstofffreien Atmosphäre unter (i) thermischen Bedingungen und/oder (ii) im Plasma zersetzt wird, und d) Silicium enthaltende Partikeln abgeschieden werden, insbesondere als amorphe Partikel umfassend Silicium mit einen Gehalt von größer gleich 30 bis 100 Gew.-% Silicium und optional auf 100 Gew.-% mit einem Gehalt an Stickstoff, Kohlenstoff, Elemente der CAS-Gruppe IIIA oder VA, bevorzugt Bor, Aluminium, Phosphor, Arsen, Selen und/oder Antimon, und optional Sauerstoff.

Dabei ist es bevorzugt, wenn die Silicium enthaltenden Partikel mit einem Fluid als amorphe Silicium enthaltende Partikel abgeschieden werden. Vorzugsweise werden die Silicium enthaltenden Partikel mit einer gasförmigen Siliciumverbindung, einem reaktiven Gas, einem Verdünnungsgas oder einem Fluid in Form einer Lösung, Emulsion, Suspension, Gel, Schaums, Aerosols oder Rauch abgeschieden werden. Erfindungsgemäße ist es bevorzugt die Partikel mit oder in einem kühleren Inertgas abzuscheiden.

In dem erfindungsgemäßen Verfahren können eingesetzt werden:

i) gasförmige oder bei erhöhter Temperatur gasförmige Siliciumverbindung umfassend Wasserstoff- und/oder Halogen-enthaltende Silane und/oder Kohlenwasserstoff-enthaltende Silane, wie Halogensilane, Chlorsilane, wie Dichlorsilan, Trichlorsilan, Tetrachlorsilan, Hexachlordisilan, Methyltrichlorsilan, Polyhalogensilane sowie reine H-Silane, wie Monosilan, Wasserstoff enthaltende Polysilane oder Polyhalogensilane und/oder mindestens ein Alkoxysilan, besonders bevorzugt ist Monosilan, Disilan, Trisilan und Gemische enthaltend mindestens eines der Silane,

ii) als Verdünnungsgas Argon, Helium, Xenon, Krypton, Wasserstoff oder ein Gemisch mindestens zwei der genannten Gase umfasst und optional

iii) als das mindestens eine reaktive Gas umfassend a) Stickstoff enthaltende Verbindungen, wie Stickstoff, $NH_3$, Alkylamine, Germanium enthaltende Verbindungen, Kohlenwasserstoffe, wie Methan, Butan und/oder Propan und optional umfasst das mindestens eine reaktive Gas b) Legierungsgase umfassend Verbindungen der Elemente der CAS-Gruppe IIIA oder VA, bevorzugt Bor, Aluminium, Phosphor, Arsen und/oder Antimon. Stickstoff ist unter den genannten Reaktionsbedingungen kein inertes Verdünnungsgas, sondern ein reaktives Gas.

Bevorzugt einsetzbare reaktive Gase umfassen auch aromatische Verbindungen, wie Toluol, insbesondere jeweils sauerstofffreie Verbindungen, mit der Maßgabe, dass sich bei der Zersetzung kein Wasser bildet. Bevorzugte reaktive Gase umfassen Kohlenwasserstoffe, wie Methan, Ethan, Propan, Butan, Gemische dieser etc., HCl, Kohlenwasserstoffe mit Stickstoff.

Die Siliciumverbindung kann eine gasförmige Verbindung sein, wie vorzugsweise Monosilan oder auch eine Verbindung, die bei erhöhter Temperatur und/oder vermindertem Druck in die Gasphase übergeht. Als gasförmige Siliciumverbindung kommen generell alle Wasserstoff enthaltenden Silane, wie Monosilan, Disilan, Trisilan und Gemische enthaltend mindestens eines der Silane in Betracht sowie Halogen und Wasserstoff oder rein Halogen enthaltende Silane und Polysilane, die auch im Gemisch in dem Verfahren umgesetzt werden können. Vorzugsweise kann die Siliciumverbindung Spuren von Si-Cl, Si-Br und/oder Halogensilane aufweisen oder eine Si-Cl enthaltende Verbindung wird in Spuren zugesetzt. Die bei erhöhter Temperatur gasförmige Siliciumverbindung kann auch Kohlenwasserstoff-enthaltende Silane umfassen. Bevorzugte Silane sind Halogensilane, Chlorsilane, wie Dichlorsilan, Trichlorsilan, Tetrachlorsilan, Hexachlordisilan, Methyltrichlorsilan, Polyhalogensilane sowie reine H-Silane wie Monosilan, Wasserstoff enthaltende Polysilane oder Polyhalogensilane und/oder mindestens ein Alkoxysilan.

Besonders bevorzugte Abkühlungsbedingungen werden nachfolgend näher erläutert. Wie vorstehend ausgeführt kann die Bildung der Pulver auf zweierleiweise erfolgen, so kann die Abkühlung und Ausbildung der Silicium enthaltenden Partikeln durch Reaktion mit kühlen reaktiven Gasen, insbesondere flüssigem Stickstoff erfolgen ggf. mit einem Gehalt an Kohlenwasserstoffen. Die Abkühlung der Zersetzungsprodukte und die Bildung der Silicium enthaltenden Partikel kann erfolgen durch Einleiten der gasförmigen Zersetzungsprodukte in flüssiges Fluide, wie Kühlmittel, bspw. flüssiges Helium oder in ein flüssiges reaktives Gas. In diesem Fall bilden sich die Silicium enthaltenden Partikel unmittelbar in dem reaktiven Gas. Eine Stabilisierung kann bspw. durch Verwendung sogenannter dem Fachmann bekannten Ionischen Flüssigkeiten verbessert werden

Eine Abkühlung kann auch durch ein Einleiten in inerte, kühle und leicht verdampfbare Flüssigkeiten oder durch Einleiten in flüssige Siliciumverbindungen oder Bor enthaltende erfolgen. Generell kann die Abscheidung erfolgen indem die Fluide zur Abscheidung eine Temperatur deutlich unterhalb 1000 °C aufweisen, vorzugsweise unter 200 °C, weiter bevorzugt unterhalb 100 °C, besonders bevorzugt unterhalb 50 °C bis -273 °C. Besonders bevorzugt erfolgt ein rasches Abscheiden durch Einstellen einer Temperaturdifferenz von größer gleich 100 °C, insbesondere größer gleich 200 °C, bevorzugt von größer gleich 500 °C, innerhalb von einer Minute, vorzugsweise innerhalb von 1000 Millisekunden, unterhalb des jeweiligen Schmelzpunktes der Silicium enthaltenden Partikel, besonders bevorzugt innerhalb von kleiner gleich 100 Millisekunden, um im Wesentlichen amorphe Partikel zu erhalten.

Besonders bevorzugte Abkühlungsbedingungen werden nachfolgend näher erläutert. Wie vorstehend ausgeführt kann die Bildung der Pulver auf zweierlei Weise erfolgen, so kann die Abkühlung und Ausbildung der Silicium enthaltenden Partikel durch Reaktion im kühlen insbesondere mit flüssigem Stickstoff gekühlten reaktiven Gasstrom, erfolgen ggf. mit einem Gehalt an Kohlenwasserstoffen Nach der Bildung der Silicium enthaltenden Partikel erfolgt die weitere Abkühlung der Zersetzungsprodukte durch Einleiten der gasförmigen Zersetzungsprodukte in flüssiges Fluide, Kühlmittel, bspw. flüssiges Helium oder in ein flüssiges reaktives Gas. In diesem Fall bindet sich auf dem Silicium enthaltenden Partikel unmittelbar das reaktiven Gas (im Sinne einer Funktionalisierung).

Eine Abkühlung des Prozessgases aus dem heißen, nicht-thermischen Plasma kann auch durch geeignete Prozessführung bspw. durch Einleiten in inerte, kühle und leicht verdampfbare Medien oder durch Einleiten in flüssige Siliciumverbindungen und/oder Bor Enthaltende erfolgen. Generell kann die Abscheidung erfolgen indem die Fluide zur Abscheidung eine Temperatur deutlich unterhalb 1000 °C aufweisen, vorzugsweise unter 200 °C, weiter bevorzugt unterhalb 100 °C, besonders bevorzugt unterhalb 50 °C bis -273 °C. Besonders bevorzugt erfolgt ein rasches Abscheiden durch Einstellen einer Temperaturdifferenz von größer gleich 100 °C, insbesondere größer gleich 200 °C, bevorzugt von größer gleich 500 °C, innerhalb von einer Minute, vorzugsweise innerhalb von 1000 Millisekunden, unterhalb des jeweiligen Schmelzpunktes der Silicium enthaltenden Partikel, besonders bevorzugt innerhalb von kleiner gleich 200 Millisekunden, um im Wesentlichen amorphe Partikel zu erhalten.

Alternativ können die amorphen Primärpartikel erhalten werden in einem im nicht-thermischen Gleichgewicht befindlichen Plasma, dessen Temperaturen bei kleiner gleich 1050 °C, vorzugsweise kleiner gleich 700 °C besonders bevorzugt bei kleiner gleich 150°C liegen. In einer anderen Variante ist die bevorzugte Prozessierung im Tieftemperaturbereich, d. h. im Bereich von 373°Kelvin und größer 0°Kelvin.

Erfindungsgemäß bevorzugt umfasst das Plasma die Bedingungen einer Gasentladung, insbesondere im nicht-thermischen Plasma.

Erfindungsgemäß eingesetzte nicht-thermische Plasmen werden beispielsweise durch eine Gasentladung oder durch Einstrahlung von elektromagnetischer Energie, wie durch Einstrahlung von Radio- oder Mikrowellen, in einer Reaktionskammer erzeugt. Die Erzeugung des Plasmas erfolgt also nicht wie bei thermischen Plasmen durch hohe Temperaturen, sondern

durch nicht-thermische Ionisationsprozesse. Dem Fachmann sind solche Plasmen bekannt. Beispielhaft sei hierzu der sogenannte Penning-Ionisationsprozess genannt.

Für die vorstehend aufgeführten Verfahren wurden im nicht-thermischen Gleichgewicht betriebene Gasentladungen verwendet. Nicht-thermisch bedeutet im erfinderischen Sinne, dass die Elektronen als energievermittelnde Spezies eine höhere Temperatur und damit eine höhere Energie (Bewegungsenergie) als die Schwerteilchen ($N$, $N_2$, $N_2^+$, $N^+$ Si, SiH, $SiH_2^+$ ... C, H, $H_2$, NH, ...) aufweisen. Diese können durch dem Fachmann bekannte bzw. geläufige Vorschaltgeräte bzw. Netzteile erzeugt werden. Das nicht-thermische Plasma weist im Allgemeinen Elektronen mit einer Energie im Bereich von 0,1 bis 100 eV, insbesondere von 1 bis 50 eV, auf und Schwerteilchen mit einer Energie im Bereich von 0,000 001 bis 10 eV, insbesondere 0,01 bis 0,1 eV.

Überraschenderweise hat es sich gezeigt, dass eine solche nicht-thermische Gasentladung sich vorteilhaft durch Dimmen (Phasenanschnittsteuerung) oder/und über Pulsweitenmodulation bzw. über die Pulsfrequenz erzeugen lässt, wobei vorteilhafterweise die Elektroden als Hohlelektroden

mit vorzugsweise porösen Stirnflächen bspw. aus Sintermetall durch eine zweidimensionale parabolische Form ausgeführt sind. Damit verteilt sich der Gasstrom gleichmäßig über die Elektrodenoberfläche. Die zweidimensionale pilzartige Oberfläche kann gemäß $F(r) = r2$ $(0,1 < r < 1,1 cm)$ beschrieben werden. Das erfindungsgemäße Verfahren wird im Allgemeinen in nicht-thermischen Plasmen durchgeführt, welche Temperaturen von 100 bis 3400 °C, vorzugsweise von 700 bis 999 °C aufweisen.

Das Plasma kann gepulst werden, vorzugsweise wird ein im Wesentlichen zylinderförmiges Plasma, insbesondere nicht-thermisches Plasma, in einem zylindrischen Bereich des Reaktionszylinders vorgesehen.

Bei den Synthesen im Plasma kann zweckmäßig mit einem Inertgas, beispielsweise einem Edelgas oder eine Mischung von Edelgasen, z. B. Argon mit geringen Anteilen an Helium, und/oder Krypton, Xenon, und/oder reaktiven Gasen wie Stickstoff, Wasserstoff, Methan, Tetrachlorkohlenstoff etc. gearbeitet werden. Andere Gasmischungen sind dem Fachmann bekannt oder können einschlägigen Lehrbüchern entnommen werden.

Eine weitere bevorzugte Ausführungsform des erfindungsgemäßen Verfahrens beinhaltet das Einleiten von Edelgas oder von Edelgasgemischen oder von Edelgas-Gasgemischen aus der Kombination Argon und/oder Helium, Krypton, Xenon als Verdünnungsgas und optional reaktiver Gase wie Stickstoff, Wasserstoff, Methan, Tetrachlorkohlenstoff etc. in das nicht-thermische Plasma, das insbesondere Temperaturen von kleiner gleich 3000 °C, vorzugsweise kleiner gleich 1900 °C aufweist.

Eine weitere bevorzugte Ausführungsform des erfindungsgemäßen Verfahrens beinhaltet das Einleiten von Edelgas oder von Edelgasgemischen als Verdünnungsgas in das nicht-thermische Plasma, das insbesondere Temperaturen von kleiner gleich 1500 °C, vorzugsweise kleiner gleich 1300 °C aufweist.

In dem Verfahren nach der Erfindung können vorzugsweise weitere bei erhöhter Temperatur gasförmige Siliciumverbindungen umgesetzt werden, umfassend Wasserstoff- und/oder Halogen-enthaltende Silane, wie H-Silane, Halogensilane, und/oder Kohlenwasserstoff-enthaltende Silane, wie Methylsilan, Methyltrichlorsilan, Dimethyldichlorsilan, Halogensilane, Chlorsilane sowie reine, hochreine, insbesondere höchstreine H-Silane, wie Monosilan und/oder Chlorsilane, wie Dichlorsilan, Trichlorsilan, Tetrachlorsilan, Hexachlordisilan, Methyltrichlorsilan, Polyhalogensilane, Wasserstoff enthaltende Polysilane, umfassend ausschließlich Wasserstoff oder Wasserstoff und Halogen. Zur Herstellung der amorphen Partikel können gleichfalls Alkoxysilane, vorzugsweise Tetramethoxysilan, Tetraethoxysilan oder gemischte Tetraalkoxysilane eingesetzt werden. Erfindungsgemäß einsetzbare Silane umfassen Silane der allgemeinen Formel I, $H_xSiCl_{4-x}$ (I), mit x unabhängig voneinander ausgewählt aus 0, 1, 2 oder 3, vorzugsweise mit x gleich 0, 1 oder 2, bevorzugt mit x gleich 0 oder 1, besonders bevorzugt mit x gleich 0, oder ein Gemisch umfassend mindestens zwei monomere Chlorsilane der Formel I, insbesondere ausgewählt aus Tetrachlorsilan, Trichlorsilan und Dichlorsilan, bevorzugt reines Tetrachlorsilan oder reines Tetrachlorsilan mit einem Gehalt an Trichlorsilan und/oder Dichlorsilan.

Bevorzugt können auch Polyperchlorsilangemische umfassend Polyperchlorsilane mit 2 bis 8 Siliciumatomen eingesetzt werden. Polychlorsilane bis 6 Siliziumatome sind einfach verdampfbar, wogegen Verbindungen ab 7 Siliciumatomen als Aerosol prozessiert werden. Besonders bevorzugt ist auch die Verwendung höhermolekularer Polychlorsilane mit mindestens drei Siliciumatomen, insbesondere mit 3 bis 8 Siliciumatomen.

Bevorzugt sind Polyperchlorsilangemische umfassend Polyperchlorsilane mit 2 bis 100 Siliciumatomen. Besonders bevorzugt ist auch die Verwendung höhermolekularer Polychlorsilane mit mindestens drei Siliciumatomen, insbesondere mit 3 bis 50 Siliciumatomen, die durch komproportionierende Reaktion $[SiCl]_n$ dargestellt werden können, wobei das $[SiCl]_n$-Molekül bevorzugt als Sechserringnetz mit n=6, sowie dessen Vielfache, vorliegen. Die Analyse erfolgt im Infraroten Spektralbereich oder durch $Si^{29}$-NMR.

Die Verwendung von Polychlorsilane entsprechend der Erfindung umfassen die homologe Reihe der Polyperchlorsilane der allgemeinen Formel II $Si_nCl_{2n+2}$, mit n grösser gleich 2, welche lineare und/oder verzweigte Ketten bilden, sowie die Polyperchlorsilane, die Ringe oder Polymere bilden, wobei die Polymere verzweigt und/oder cyclisch sein können, mit der idealisierten Formel III $Si_nCl_{2n}$, mit n grösser gleich 3, als auch die Siliciumchloride mit geringerem Chlorgehalt der idealisierten Formel IV SiCl$_{1,5}$. Besonders bevorzugt gelten als Polychlorsilane Verbindungen der allgemeinen Formel II Si$_n$Cl$_{2n+2}$, mit n grösser gleich 2, insbesondere mit n grösser gleich 2 bis 100, vorzugsweise mit n grösser gleich 2 bis 50, vorzugsweise jeweils unabhängig mit n grösser gleich 2, 3, 4, 5, 6, 7, 8, 9 oder 10, bevorzugt 2 bis 8, besonders bevorzugt mit n gleich 2 oder 3, wobei sie lineare als auch verzweigte Ketten bilden können, und Verbindungen der allgemeinen Formel III, die Ringe oder Polymere bilden mit Si$_n$Cl$_{2n}$, mit n grösser gleich 3, insbesondere mit n grösser gleich 4 bis 100, insbesondere mit n grösser gleich 4 bis 50, besonders bevorzugt jeweils unabhängig mit n grösser gleich 4, 5, 6, 7, 8, 9 oder 10, als auch Polychlorsilane mit geringerem Chlorgehalt gemäss der allgemeinen Formel IV mit Si$_n$Cl$_{1,5n}$, mit n grösser gleich 4 oder 5, insbesondere mit n grösser gleich 6 bis 200, vorzugsweise mit n grösser gleich 8 bis 100. Besonders bevorzugt wird ein Polychlorsilan (PCS) eingesetzt, insbesondere Octachlortrisilan oder ein Octachlortrisilan in einer Mischung mit höhermolekularen Polychlorsilanen, vorzugsweise Polyperchlorsilanen, wobei das Polychlorsilan insbesondere einen Gehalt an Octachlortrisilan von 20 bis 99,9999 Gew.-% aufweist, vorteilhaft mit einem vorgenannten Verunreinigungsprofil. Es kann auch ein gelöstes Polysilan und/oder Polychlorsilan beispielsweise in einem reaktiven Lösemittel, wie Kohlenwasserstoff als Flüssigkeit, Sirup, Paste, Creme, Dispersion, Emulsion, eingesetzt werden, wobei das hochreine Lösemittel vor der Zersetzung zu einem reaktiven Gas verdampft wird. Somit gelten auch reaktive verdampfbare Lösemittel als reaktive Gase.

Das Verdünnungsgas kann vorzugsweise mit der gasförmigen Silicium-Verbindung und/oder dem reaktiven Gas vor dem Einbringen in den Reaktionszylinder gemischt werden. Alternativ wird das inerte Verdünnungsgas zur Abscheidung der Partikel verwendet werden. Die Zersetzung der Silicium-Verbindung und optional des reaktiven Gases kann auch durch Überführen der in einen Hochvakuum-Bereich erfolgen. Als ein Hoch-Vakuum wird ein Vakuum kleiner gleich 0,01 bar, insbesondere kleiner gleich 0,001 bar, kleiner gleich 0,0001 bar erachtet.

Der Druckbereich liegt üblicherweise bei 0,001 mbar bis 50 bar, insbesondere bei 1 mbar bis 10 bar, bevorzugt bei 10 mbar bis 5 bar. Je nach dem gewünschten Zersetzungs- und/oder Legierungs- und/oder Beschichtungsprodukt und, um die Bildung von Kohlenstoff enthaltenden Prozessgasen zu minimieren, kann das Verfahren auch in einem Druckbereich von 1 bis 50 bar erfolgen, bevorzugt bei 2 bis 50 bar, besonders bevorzugt bei 5 bis 50 bar. Dabei weiß der Fachmann, dass der zu wählende Druck ein Kompromiss zwischen Gasabführung, Agglomerierung und Reduktion der Kohlenstoff enthaltenden Prozessgase ist.

Zur Durchführung des Verfahrens ist es zusätzlich zu den vorgenannten Merkmalen weiter bevorzugt, wenn die Gasentladung ein nicht-thermisches Plasma ist, weiter bevorzugt erfolgt die Anregung der Gasentladung durch ein Generator wie er auch in einem Ozonisator Anwendung findet. Für die Definition des nicht-thermischen Plasmas wird auf die einschlägige Fachliteratur verwiesen, wie beispielsweise auf *"Plasmatechnik: Grundlagen und Anwendungen - Eine Einführung; Autorenkollektiv, Carl Hanser Verlag, München/Wien; 1984, ISBN 3 446-13627-4"*.

Der spezifische Leistungseintrag beträgt von 000,1 bis 1000 W/cm$^2$. Besonders bevorzugt beträgt der spezifische Energieeintrag von 0,1 bis 100 Ws/cm$^2$ im beispielhaften Fall mit Spaltweiten (GAP) von beispielhaft 1 mm. Dabei ist es weiter bevorzugt, wenn der spezifische Energieeintrag mittels phasengenauer Momentanleistungsmessung mit einer Bandbreite von mindestens 250 kHz durchgeführt wird. Die Bestimmung der Momentanleistung erfolgt in einer normierten Anordnung mit 50 cm$^2$ Entladungsfläche. Der Energieeintrag zur Ausbildung des nicht-thermischen Plasmas erfolgt vorzugsweise in der Art, dass sich in dem sich ausbildenden Plasma möglichst homogene Bedingungen für die Umsetzung der Silane sowie der C, N und/oder Ge etc. enthaltenen Verbindungen ausbilden, dabei ist es besonders bevorzugt, wenn das nicht-thermische Plasma bei einer Spannung betrieben wird, bei der die Entladung die gesamte Elektrodenfläche bedeckt.

Entsprechend einer bevorzugten Alternative werden die abgeschiedenen Partikel an ggf. oberflächlich vorhandenen Sauerstoff-Atomen und/oder Chlor-Atomen organofunktionalisiert. Vorzugsweise werden die Partikel an der Oberfläche der Partikel durch Reaktion mit einer reaktiven organofunktionellen Gruppe des Silans modifiziert. Die Modifizierung kann über Komplexierung oder eine Ausbildung kovalenter Bindungen erfolgen. Generell können die Silicium enthaltenden Partikeln zumindest teilweise mit einem organofunktionellen Silan modifiziert werden. Organofunktionelle Silane umfassen Silane mit ungesättigten Kohlenwasserstoffresten, Halogenfunktionalisierte Silane, wie vorzugsweise Halogenalkylsilane, wie Monochlortrimethylsilan, Halogenalkoxysilane, wie Monochlortrialkoxysilan, Alkylenalkoxysilane, Alkylenhalogensilane, Amino-funktionelle Silane, wie Aminopropyltriethoxysilan, Aminopropyltrialkylsilan, sowie organofunktionelle Silane. Organofunktionelle Silane umfassen auch organisch funktionalisierte Siliciumverbindungen und Organosiloxanen.

Gegenstand der Erfindung sind auch UV-Schutz Zusammensetzung enthaltend amorphe Silicium enthaltende Partikel mit einen Gehalt von größer gleich 30 bis 100 Gew.-% Silicium und auf 100 Gew.-% mit einem Gehalt an Stickstoff, Kohlenstoff, Elemente der CAS-Gruppe IIIA oder VA, bevorzugt Bor, Aluminium, Phosphor, Arsen und/oder Antimon, und optional Sauerstoff, wobei die mittlere Primärpartikelgröße 1 bis 500 nm beträgt, die Partikel vorzugsweise als Cluster von Primärpartikeln vorliegen. Die Zusammensetzung liegt vorzugsweise als Formulierung vor, insbesondere als UV-Schutz-Formulierung für technische Anwendungen und/oder technische Materialien, vorliegt und mindestens ein Hilfsmittel umfasst. Die Formulierung kann als Pulver, Dispersion, Suspension, Aerosol, eingebettet in einer Schicht, beispielsweise als Folie, einem Coating etc. vorliegen.

Eine erfindungsgemäße Formulierung umfasst vorzugsweise mindestens ein Hilfsmittel, Additiv oder weitere übliche Formulierungsbestandteile.

Da die erfindungsgemäßen Partikel inert sind, eignen sie sich wegen ihres Transmissionsverhaltens besonders gut als UV-Schutzmittel, denn die erfindungsgemäßen Partikel, wie die SiC-Pulver, können transparent vorliegen und vorteilhaft um 300 nm +/- 80 nm keine nennenswerte Transmission aufweisen.

Als im Wesentlichen amorph gilt ein Pulver, das röntgenamorph ist. Als röntgenamorph gilt vorzugsweise ein Pulver mit einem Kristallinität kleiner 2 %. Die Kristallinität, die im Rahmen der Erfindung auch *"Kristallinitätsgrad"* genannt wird, kann mittels XRPD über die folgende Formel ermittelt werden:

$$(100 \times A)/(A + B - C) = \text{Kristallinität in \%}$$

Dabei ist A die gesamte Peakfläche der Reflexe der kristallinen Bestandteilen des Diffraktogramms, B die gesamte Fläche unterhalb der Peakfläche A, C die luftstreuend-, fluoreszierend- und gerätebedingte Untergrundfläche.

Unterhalb der schmalen Reflexe der Si-Phase kann die Peakfläche A einen Untergrund aufweisen. Die Untergrundfläche C wurde anhand der XRD-Diagramme des Si-Referenzstandards NIST 640 (Si-Standard = 100% Kristallinität) ermittelt. Fläche B entspricht einem eingelegten Untergrundverlauf sowie dem konstanten Untergrund C. Berechnung (HighScore Plus Software).

In röntgenamorphen Pulvern gibt es im XRPD keine scharfen, sondern nur wenige diffuse Interferenzen bei kleinen Beugungswinkeln. Stoffe mit einem derartigen Röntgenbeugungsdiagramm bezeichnet man als *röntgenamorph.* Im Falle der Kristallinität liegt ein anisotroper, homogener Körper vor, der eine dreidimensional periodische Anordnung der Bausteine besitzt und ein XRPD mit klar definierten auflösbaren Reflexen besitzt.

Die Partikelgrößenbestimmung als auch die Bildung von Clustern, wie Agglomeraten oder das Vorliegen von Primärpartikeln kann analytisch mit nachfolgenden Methoden bestimmt werden. Die Bestimmung der Teilchengröße kann u.a. mittels Siebanalyse, TEM (Transelektronenmikroskopie), REM (Rasterkraftelektronenmikroskopie) oder Lichtmikroskopie erfolgen.

**Ausführungsbeispiele**

Figur 1a zeigt eine TEM-Aufnahme der erfindungsgemäßen Silicium enthaltenden Partikel.

Figur 1b zeigt das UV-Vis-Spektren zum erfindungsgemäßen Produkt 6 (P6) und in der Gegenüberstellung ein typisches Vergleichsprodukt TiO2 Typ P25 (Nr. 5 in Figur 1b) von Evonik. Die nachfolgende Tabelle 1 offenbart exemplarisch die gemessenen Absorptionen (Absorption: A, rel. Einheit) in Abhängigkeit von der Wellenlänge ($\lambda$ [nm]) und in Abhängigkeit von der Partikelgröße der Proben Nr. 1 bis 5. Der Pfeil zeigt in die Richtung wachsender mittlerer Partikelgröße.

Tabelle 1: UV/Vis bis IR Spektren (Figur 1b):

| Spektrum Probe Nr. | | | | | |
|---|---|---|---|---|---|
| **1** | **4** | **3** | **5** | **2** | **[$\lambda$ [nm]** |
| 1,7 | 2,4 | 2,6 | 2,6 | 1,6 | 200 |
| 2,7 | 1 | 1,4 | 1,07 | 2,3 | 250 |
| 2,3 | 1 | 1,3 | 1,05 | 2,1 | 300 |
| 2,7 | 1 | 1,25 | 1,05 | 2,1 | 350 |
| 2,3 | 1,03 | 1 | 0,9 | 1,5 | 400 |
| 1,4 | 1,02 | 0,6 | 0,8 | 0,8 | 450 |
| 0,75 | 0,97 | 0,4 | 0,72 | 0,55 | 500 |
| 0,57 | 0,92 | 0,3 | 0,65 | 0,38 | 550 |
| 0,5 | 0,88 | 0,24 | 0,6 | 0,26 | 600 |
| 0,35 | 0,84 | 0,2 | 0,56 | 0,22 | 650 |
| 0,3 | 0,8 | 0,16 | 0,53 | 0,18 | 700 |
| 0,25 | 0,79 | 0,12 | 0,5 | 0,14 | 750 |
| 0,22 | 0,76 | 0,1 | 0,48 | 0,12 | 800 |
| 0,22 | 0,73 | 0,1 | 0,45 | 0,12 | 850 |
| 0,21 | 0,67 | 0,09 | 0,42 | 0,11 | 900 |
| 0,18 | 0,68 | 0,08 | 0,4 | 0,1 | 950 |
| 0,16 | 0,66 | 0,06 | 0,39 | 0,1 | 1000 |
| 0,14 | 0,65 | 0,05 | 0,38 | 0,1 | 1050 |

Die Spektren 1, 2, 3, 4, und 5 der Proben zeigen für den Wellenlängenbereich oberhalb von 650 nm für die Proben 4 und 5 eine relativ konstante Absorption, wobei die Absorption der drei Proben Nr. 1, 2 und 3 mit den kleinsten mittleren Partikelgrößen weiter kontinuierlich abnimmt.

Die Zuordnung der Spektren 1, 2, 3, 4 und 5 der Figur 1b zu den Proben ist von großen zu kleinen Werten:

1: Probe Nr. 1: Si, $d_{50}$ = 5-25 nm (2013048401)
2: Probe Nr. 2: Si, $d_{50}$ = 10-20 nm (20131120)
3: Probe Nr. 3: Si, $d_{50}$ = 20-70 nm
4: Probe Nr. 4: Si, $d_{50}$ = 20-100 nm
5: Probe Nr. 5: TiO$_2$-P25

Figur 2 stellt die Brechzahl von Silicium in Abhängigkeit von der Wellenläng (λ [nm]) dar, Spektrum e: Brechzahl Silicium, Linie f: Brechzahl n = 4.

Figuren 3a und 3b zeigen TEM-Aufnahmen der Probe P6 in unterschiedlicher Vergrößerung (Figur 3 (100000 : 1, 200 nm) und Figur 3b (600000 : 1, 50 nm).

Figur 4 zeigt die Größenverteilung der Cluster. Es stellen dar Nr. 1 (20131007 Si P6 5), Nr. 2 (20131007 Si P6 3), Nr. 3 (20131007 Si P6 2), Nr. 4 (20131007 Si P6 4), Nr. 5 (20131007 Si P6 1),

Figur 5 zeigt das UV-Vis-Absorptionsverhalten einer Referenzprobe sowie von Si unterschiedlicher Primärpartikel- und unterschiedlicher Clustergrößen. In Richtung des Pfeils (Zunahme Partikelgröße) nimmt die Absorption mit zunehmender Partikelgröße zu im Wellenlängenbereich von oberhalb 400 nm bis weit über 100 nm zu. Zuordnung der Spektren in Figur 5: Spektrum d: 20131007-P6/2mm, Spektrum b: Ti0$_2$-P25, Spektrum a: 20130912-P4: Probe 4, Spektrum c: 20130912-P3: Probe 3

Figur 6 zeigt eine TEM Aufnahme der Partikel der Probe P4.

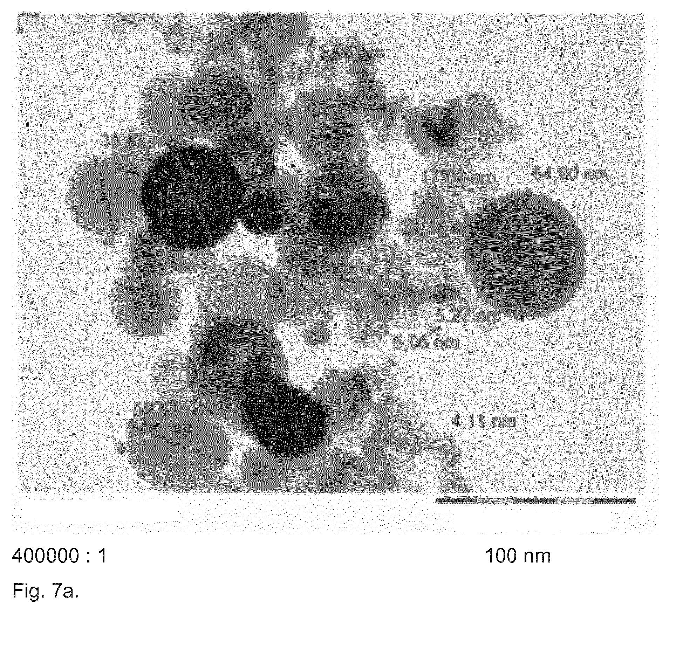

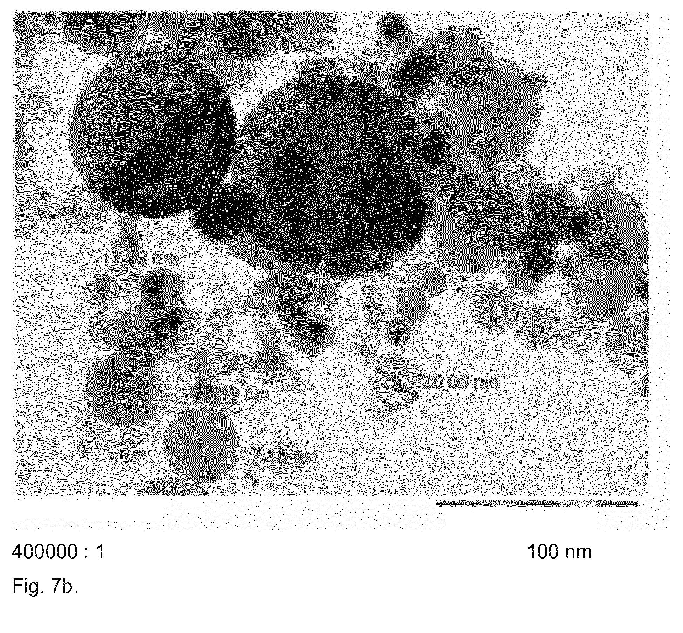

Die Fig. 7a zeigt eine TEM Aufnahme der Probe 3, die aus einem Free-Space Reaktor erhalten wurde. Die Fig. 7b zeigt eine TEM Aufnahme der Probe 4. Beide Proben waren amorph.

Figur 8a zeigt eine TEM Aufnahme der Produkte des Ausführungsbeispiels 2a und Figur 8b zeigt eine TEM Aufnahme des Produktes aus Ausführungsbeispiel 2b.

Alle Liter Angaben sind in der Einheit Normliter. Der Plasmareaktor wurde bei 0,4 kW und ca. 16 kHz mit Hochspannungspulsen mit einer Halbwertsbreite von t(50) 500 ns und einem Massestrom von 40 NL/min betrieben. Für die nachstehend genannten Plasma-Verfahren wurden im nicht-thermischen Gleichgewicht betriebene Gasentladungen verwendet.

Der in den Beispielen eingesetzte Free-Space Reaktor, abgekürzt "FSR", wies eine tangentiale Wandströmung auf. Der FSR war mit einer Anordnung zur Temperatur-Messung im Reaktor ausgestattet. Die Geometrie eines bevorzugten Reaktors ist nachfolgend wiedergegeben. Das Reaktor-Rohr wies einen Außendurchmesser von 36,1 mm auf, und einen Innendurchmesser von 33,1 mm. Die Heizzone war auf eine Länge von 700 mm bei einer Temperatur von 1300 °C vorgesehen.

Das Abschirmrohr für die Temperaturmess-Lanze wies einen Außendurchmesser von 6,7 mm, einen Innendurchmesser von 3,7 mm, und einen Messfühler für eine Messung hoher Temperaturen bis zu 2000 °C auf. Für eine Zirkulargasströmung im FSR war eine tangentiale Zuführung vorgesehen. Die nachfolgende Tabelle 2 stellt die Temperaturkurve im Reaktor an verschiedenen Messpunkten dar.

**Tabelle 2:** Temperaturkurve in Abhängigkeit vom Messpunkt (x-Wert in mm) im Rohrreaktor

| Messpunkt | X-Wert (mm) | Temperaturwert |
|---|---|---|
| M1 | -50 | RT |
| M2 | 0 | 595 |
| M3 | 50 | 926 |
| M4 | 100 | 1078 |
| M5 | 150 | 1149 |
| M6 | 200 | 1199 |
| M7 | 250 | 1227 |
| M8 | 300 | 1239 |
| M9 | 350 | 1238 |
| M10 | 400 | 1222 |
| M11 | 450 | 1186 |
| M12 | 500 | 1120 |
| M13 | 550 | 929 |
| M14 | 600 | 553 |
| M15 | 650 | RT |

**Ausführungsbeispiel 1:** Gewinnung von amorphen Silicium Partikeln in hochreiner Form in einem Free-Space Reaktor. Es wird Monosilan mit in einer H$_2$-Matrix (60 Vol.-%) zersetzt. Der Wasserstoff wird als Wärmeüberträger und als Verdünnungsgas verwendet. Die Verweilzeit beträgt von 100 bis 500 Millisekunden in einem Rohrreaktor mit einer Länge von 50 cm.

Die Abkühlung und Abtrennung des amorphen Silicium erfolgt in einem Fluid. Vorliegend wurden die Zersetzungsprodukte durch flüssiges Paraffin geleitet. Die gebildeten amorphen Silicium Primärpartikel können im Paraffin stabilisiert werden und liegen je nach Herstellbedingungen und Konzentration in Form von aneinandergewachsenen Primärpartikel in Clustern vor.

Die Partikel haben je nach den konkreten Verfahrensbedingungen unterschiedliche Primärpartikelgrößen. Die Verfahrensbedingungen waren

(a) Einsetzen einer Gasmischung aus

- 2 Normliter (NL/Min) Argon, 1 NL/Min Argon mit 5 Gew.-% SiH$_4$ zur Herstellung der Proben 3a und 3b.
- Für die Probe 3a wurde eine Verweilzeit von 100 Millisekunden, und für die Probe 3b eine Verweilzeit von 500 Millisekunden gewählt.

(b) Einsetzen einer Gasmischung aus

- 2 NL/Min Argon und 2 NL/Min Argon mit 5 Gew.-% SiH$_4$ zur Herstellung der Probe 4.

- Für die Probe 4 wurde eine Verweilzeit von 400 Millisekunden in dem Rohrreaktor gewählt.

Die bei (a) erhaltene Probe 3a wies eine Primärpartikelgröße $d_{50}$ von 20 bis 25 nm auf. Bei der Probe 3b lag diese Größe von 50 bis 55 nm.

Die bei (b) erhaltene Probe 4 wies eine Primärpartikelgröße $d_{50}$ von 35 bis 40 nm auf.

Die Absorption der in einer Emulsion in Ethanol dispergierten amorphen Siliciumpartikel der Probe 3 mit einer Partikelgröße von etwa 45 nm und Clustergrößen von etwa 350 nm oder weniger betrug im Bereich von 400 bis 1050 nm unter 1.

**Ausführungsbeispiele 2a und 2b: Plasma**

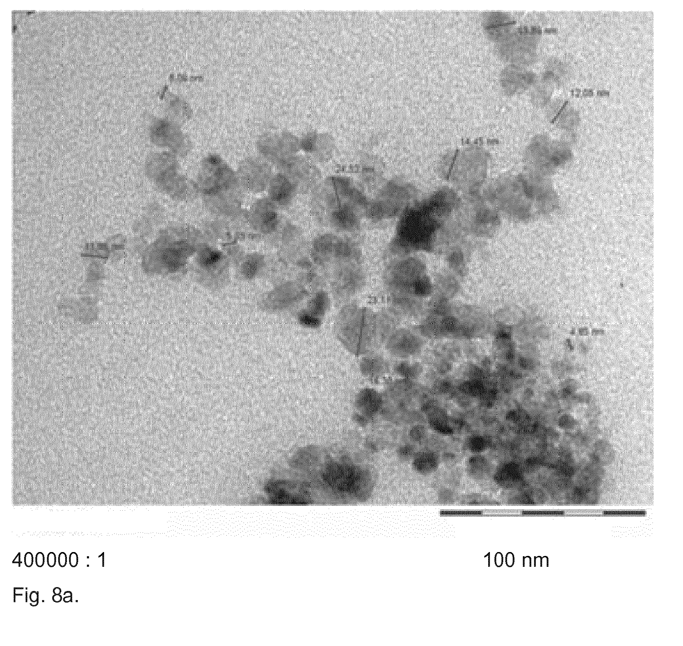

(2a) Monosilan wurde in einem im nicht-thermischen Gleichgewicht befindlichen Argon Plasma umgesetzt. Die erhaltene mittlere Primärpartikelgröße lag bei 5 bis 10 nm. Das erhaltene Umsetzungsprodukt wurde in verdünntem Chloroform dispergiert. Eine TEM Aufnahme dieser Dispersion zeigt Fig. 8a.

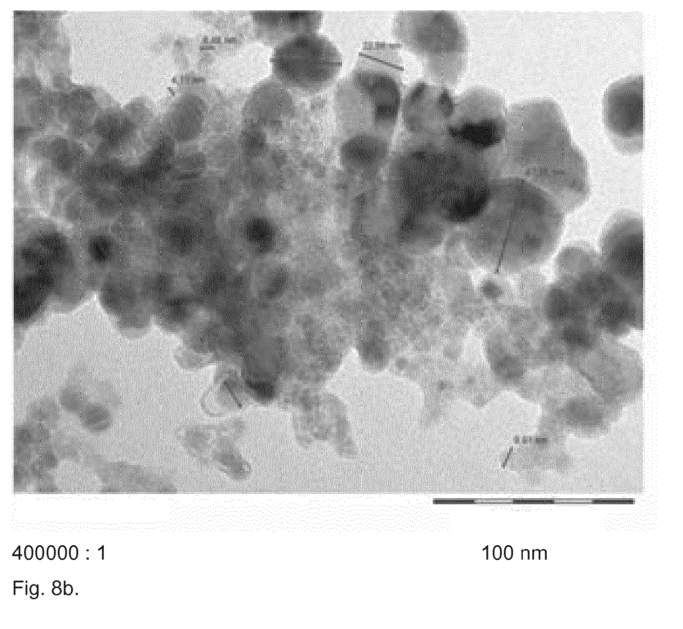

(2b) Wie Versuch (2a), jedoch mit verlängerten Kontaktzeiten. Man erhielt mittlere Primärpartikelgrößen bei etwa 30 nm. Die TEM Aufnahme der Dispersion in verdünntem Chloroform zeigt Fig. 8b.

**Patentansprüche**

1. Verwendung von Silicium enthaltenden Partikeln, **dadurch gekennzeichnet, dass** die Partikel zum Schutz technischer Materialien vor elektromagnetischer Strahlung im Wellenlängenbereich von 10 bis 1500 nm verwendet werden.

2. Verwendung nach Anspruch 1, **dadurch gekennzeichnet, dass** die Partikel

a) zur Absorption von elektronmagnetischer Strahlung im Wellenlängenbereich von 10 nm bis 450 nm verwendet werden,
b) als UV-Schutz für technische Materialien, Oberflächen, Bauteile, elektrooptische Schichten, elektrooptischer Bauteile verwendet werden.

3. Verwendung nach Anspruch 1 oder 2, **dadurch gekennzeichnet, dass** die Silicium enthaltenden Partikel einen Gehalt von größer gleich 40 bis 100 Gew.-% Silicium aufweisen und auf 100 Gew.-% einen Gehalt an Stickstoff, Kohlenstoff und/oder Elemente der CAS-Gruppe IIIA oder VA aufweisen, bevorzugt Bor, Aluminium, Phosphor, Arsen und/oder Antimon, und optional Sauerstoff, wobei die Primärpartikelgröße von 1 bis 500 nm beträgt.

4. Verwendung nach einem der Ansprüche 1 bis 3, **dadurch gekennzeichnet, dass** die Silicium enthaltenden Partikel im Wesentlichen amorphe Partikel sind.

5. Verwendung nach einem der Ansprüche 1 bis 4, **dadurch gekennzeichnet, dass** die Silicium enthaltenden Partikel im Wesentlichen amorphe Partikel aus Silicium, Siliciumcarbid, Siliciumnitrid ($Si_3N_4$), SiC-Si, SiGe, SiGe:C oder Gemischen dieser umfassen.

6. Verwendung nach einem der Ansprüche 1 bis 5, **dadurch gekennzeichnet, dass** die Silicium Partikel eine Kristallinität von kleiner gleich 2 % aufweisen.

7. Verwendung nach einem der Ansprüche 1 bis 5, **dadurch gekennzeichnet, dass** die Silicium enthaltenden Partikel

a) eine mittlere Primärpartikelgröße von im Mittel $d_{50}$ = 1 nm bis 200 nm aufweisen,
b) eine mittlere Primärpartikelgröße von im Mittel $d_{50}$ = 1 bis 10 nm,
c) eine mittlere Primärpartikelgröße von im Mittel $d_{50}$ = 5 bis 10 nm, und/oder
d) eine mittlere Primärpartikelgröße von im Mittel $d_{50}$ = 16 bis 40 nm oder Gemische dieser mit Fraktionen der vorgenannten Primärpartikelgrößen.

8. Verwendung nach einem der Ansprüche 1 bis 7, **dadurch gekennzeichnet, dass** die Partikel zu 40 bis 100 Gew.-% Silicium umfassen und auf 100 Gew.-% einen Gehalt an Stickstoff, Kohlenstoff und optional Sauerstoff aufweisen, und optional transparent sind.

9. Verwendung nach einem der Ansprüche 1 bis 8, **dadurch gekennzeichnet, dass** die Partikel mit einer mittleren Primärpartikelgröße $d_{50}$ von 1 bis 50 nm und optional einer mittleren Clustergröße von 50 bis 150 nm, insbesondere 50 bis 100nm, im Mittel eine doppelt so große Absorption im Wellenlängenreich von 200 bis 400 nm aufweisen im Vergleich zur Absorption im Wellenlängenbereich von 500 bis 750 nm.

10. Verwendung nach einem der Ansprüche 1 bis 9, **dadurch gekennzeichnet, dass** die Partikel die nachfolgende Absorptionscharakteristika von elektronmagnetischer Strahlung aufweisen

a) mit einer Primärpartikelgröße von 10 bis 50 nm, insbesondere mit $d_{50}$ um 35 nm, ist die Absorption in Wellenlängenbereich von 250 bis 400 im Vergleich zur Absorption im Wellenlängenbereich von 400 bis 750 nm mit einer Abweichung von +/- 40 % gleich, und/oder

b) mit einer Primärpartikelgröße von 10 bis 50 nm, insbesondere mit $d_{50}$ um 25 nm, ist das Verhältnis der Absorption in Wellenlängenbereich von (i) 250 bis 400 im Vergleich zur Absorption bei einer Wellenlänge von (ii) 550 nm etwa (i) zu (ii) von 2 : 1 bis 8 : 1 mit einer Abweichung von +/- 40 %, und/oder

c) mit einer Primärpartikelgröße von 10 bis 50 nm, insbesondere mit $d_{50}$ um 25 nm, ist das Verhältnis der Absorption in Wellenlängenbereich von (i) 250 bis 400 im Vergleich zur Absorption bei einer Wellenlänge von (ii) 500 nm bei etwa (i) zu (ii) von 1,5 : 1 bis 8 : 1 mit einer Abweichung von +/- 30 %, und/oder

d) mit einer Primärpartikelgröße von 10 bis 50 nm, insbesondere mit $d_{50}$ um 25 nm, ist das Verhältnis der Absorption in Wellenlängenbereich von (i) 250 bis 350 nm im Vergleich zur Absorption bei einer Wellenlänge von (ii) 450 nm etwa (i) zu (ii) von 2 : 1 bis 4 : 1 mit einer Abweichung von +/- 30 %, und/oder

e) mit einer Primärpartikelgröße von 10 bis 50 nm, insbesondere mit $d_{50}$ um 25 nm, ist das Verhältnis der Absorption bei einer Wellenlänge von (i) 250 nm im Vergleich zur Absorption bei einer Wellenlänge von (ii) 450 nm bei etwa (i) zu (ii) von 2 : 1 bis 4 : 1, mit einer Abweichung von +/- 30 %, und/oder

f) mit einer Primärpartikelgröße von 10 bis 50 nm, insbesondere mit $d_{50}$ um 25 nm, ist das Verhältnis der Absorption bei einer Wellenlänge von (i) 350 nm im Vergleich zur Absorption bei einer Wellenlänge von (ii) 450 nm bei etwa (i) zu (ii) von 2 : 1 bis 3 : 1, mit einer Abweichung von +/- 30 %.

11. Verwendung, insbesondere nach einem der Ansprüche 1 bis 10,
**dadurch gekennzeichnet, dass**
Silicium enthaltende Partikel zur Erhöhung der Brechzahl eines Materials verwendet werden.

12. Verwendung nach einem der Ansprüche 1 bis 11,
**dadurch gekennzeichnet, dass**

a) die Silicium enthaltenden Partikel einen Gehalt an Silicium von 90 bis 100 Gew.-% aufweisen und eine Brechzahl (n) von größer gleich 3 im Wellenlängenbereiche von 500 bis 2500 nm und/oder größer gleich 4,0 bei einer Wellenlänge von 200 bis 500 nm aufweisen, oder
b) die Silicium enthaltenden Partikel ausgewählt aus SiC-Partikeln und Partikeln mit einem Gehalt an Silicium und Kohlenstoff mit Silicium 70 bis 90 Gew.-% und 10 bis 30 Gew.-% Kohlenstoff, wobei die Partikel eine Brechzahl (n)

i) von größer gleich 2,5 bis kleiner gleich 1000 nm,
ii) von größer gleich 2,75 bis kleiner gleich 500 nm,
iii) von größer gleich 3,0 bis kleiner gleich 230 nm, und/oder
iv) von größer gleich 3,5 bis kleiner gleich 50 nm aufweisen.

13. Verwendung nach einem der Ansprüche 1 bis 12,
**dadurch gekennzeichnet, dass**
die technischen Materialien in der Masse und/oder mittels einer Beschichtung vor elektromagnetischer Strahlung geschützt werden, als UV-Schutz elektrooptischer Schichten, als UV-Schutz elektrooptischer Bauteile und/oder zur Verwendung als Beschichtung.

14. Verwendung von Silicium enthaltenden Partikeln umfassend Primärpartikel von 1 bis 100 nm und optional Cluster dieser Primärpartikel, mit einem Gehalt an Silicium von größer gleich 90 Gew.-% bis 100 Gew.-% als biologisch verträglicher UV-Schutz und/oder als biologisch abbaubarer UV-Schutz.

15. Verfahren zur Herstellung von Silicium enthaltenden Partikeln, insbesondere zur Verwendung nach einem der Ansprüche 1 bis 14, indem

a) mindestens eine gasförmige oder bei erhöhter Temperatur gasförmige Siliciumverbindung,
b) optional in Gegenwart mindestens eines unter den Reaktionsbedingungen reaktiven Gases oder einer Mischung von reaktiven Gasen,
c) in Gegenwart eines Verdünnungsgases in einer im Wesentlichen sauerstofffreien Atmosphäre unter (i) thermischen Bedingungen und/oder (ii) im Plasma zersetzt wird, und
d) Silicium enthaltende Partikeln abgeschieden werden.

16. Verfahren nach Anspruch 14,
**dadurch gekennzeichnet, dass** die

i) gasförmige oder bei erhöhter Temperatur gasförmige Siliciumverbindung umfasst Wasserstoff- und/oder Halogen-enthaltende Silane und/oder Kohlenwasserstoff-enthaltende Silane, wie Halogensilane, Chlorsilane, wie Dichlorsilan, Trichlorsilan, Tetrachlorsilan, Hexachlordisilan, Methyltrichlorsilan, Polyhalogensilane sowie reine H-Silane, wie Monosilan, Wasserstoff enthaltende Polysilane oder Polyhalogensilane und/oder mindestens ein Alkoxysilan,

ii) das Verdünnungsgas Argon, Helium, Xenon, Krypton, Wasserstoff oder ein Gemisch mindestens zwei der genannten Gase umfasst und optional

iii) das mindestens eine reaktive Gas umfasst a) Stickstoff enthaltende Verbindungen, wie Stickstoff, $NH_3$, Alkylamine, Germanium enthaltende Verbindungen, Kohlenwasserstoffe, wie Methan, Butan und/oder Propan und optional umfasst das mindestens eine reaktive Gas b) Legierungsgase umfassend Verbindungen der Elemente der CAS-Gruppe IIIA oder VA, bevorzugt Bor, Aluminium, Phosphor, Arsen und/oder Antimon.

**17.** UV-Schutz Zusammensetzung enthaltend amorphe Silicium enthaltende Partikel nach Anspruch 14 oder 15,

oder amorphe Silicium enthaltende Partikel mit einen Gehalt von größer gleich 30 bis 100 Gew.-% Silicium und auf 100 Gew.-% mit einem Gehalt an Stickstoff, Kohlenstoff, Elemente der CAS-Gruppe IIIA oder VA, bevorzugt Bor, Aluminium, Phosphor, Arsen und/oder Antimon, und optional Sauerstoff, wobei

die mittlere Primärpartikelgröße 1 bis 500 nm beträgt, die Partikel vorzugsweise als Cluster von Primärpartikeln vorliegen.

**18.** Zusammensetzung nach Anspruch 16,
**dadurch gekennzeichnet, dass**
sie als Formulierung, insbesondere als UV-Schutz-Formulierung für technische Anwendungen und/oder technische Materialien, vorliegt und mindestens ein Hilfsmittel umfasst.

Fig. 1a

Fig. 1b

Fig. 2.

100000 : 1                              200 nm

Fig. 3a.

600000 : 1                          50 nm

Fig. 3b.

Fig. 4.

Fig. 5.

200000 : 1                                    200 nm

Fig. 6.

400000 : 1                                                  100 nm

Fig. 7a.

400000 : 1                                                                    100 nm

Fig. 7b.

400000 : 1                                          100 nm

Fig. 8a.

400000 : 1                                        100 nm

Fig. 8b.

**Europäisches Patentamt**
**European Patent Office**
**Office européen des brevets**

# EUROPÄISCHER RECHERCHENBERICHT

Nummer der Anmeldung

EP 14 19 5301

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 96/16114 A1 (KUEHNLE MANFRED R [US]) 30. Mai 1996 (1996-05-30) * Seite 22, Zeile 17 - Seite 23, Zeile 10; Abbildungen 14,16-23, 26-33 * * Seite 29, Zeilen 25-32 * * Seite 30, Zeile 21 - Seite 33, Zeile 8 * ----- | 1-14,17, 18 | INV. A61K8/25 C01B33/02 C01B33/027 C01B33/029 |
| X | WO 2013/025707 A1 (DOW CORNING [US]; DEHTIAR MAX [US]; FISHER PAUL [US]; GAVE MATTHEW A []) 21. Februar 2013 (2013-02-21) * Absätze [0033], [0034] * ----- | 15,16 | |

**RECHERCHIERTE SACHGEBIETE (IPC)**

A61K
C01B

Der vorliegende Recherchenbericht wurde für alle Patentansprüche erstellt

| Recherchenort | Abschlußdatum der Recherche | Prüfer |
|---|---|---|
| München | 18. Mai 2015 | Uhl, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 19 5301

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-05-2015

| Im Recherchenbericht angeführtes Patentdokument | Datum der Veröffentlichung | Mitglied(er) der Patentfamilie | Datum der Veröffentlichung |
|---|---|---|---|
| WO 9616114 A1 | 30-05-1996 | AU 4238296 A | 17-06-1996 |
| | | CA 2207587 A1 | 30-05-1996 |
| | | EP 0800550 A1 | 15-10-1997 |
| | | JP H10512305 A | 24-11-1998 |
| | | US 5527386 A | 18-06-1996 |
| | | WO 9616114 A1 | 30-05-1996 |
| WO 2013025707 A1 | 21-02-2013 | EP 2745341 A1 | 25-06-2014 |
| | | JP 2014528893 A | 30-10-2014 |
| | | KR 20140052015 A | 02-05-2014 |
| | | TW 201316597 A | 16-04-2013 |
| | | US 2014220347 A1 | 07-08-2014 |
| | | WO 2013025707 A1 | 21-02-2013 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **A.T. BELL.** Fundamentals of Plasma Chemistry. Wiley, 1974

- Plasmatechnik: Grundlagen und Anwendungen - Eine Einführung; Autorenkollektiv. Carl Hanser Verlag, 1984, vol. 3, 446-13627, 4